(19) **Europäisches Patentamt**
**European Patent Office**
**Office européen des brevets**

(11) **EP 2 000 541 A1**

(12) **EUROPEAN PATENT APPLICATION**
published in accordance with Art. 153(4) EPC

(43) Date of publication:
**10.12.2008 Bulletin 2008/50**

(51) Int Cl.:
*C12P 21/08* [(2006.01)]    *A61K 39/395* [(2006.01)]
*C07K 16/28* [(2006.01)]    *C12N 15/00* [(2006.01)]

(21) Application number: **06767958.9**

(22) Date of filing: **06.07.2006**

(86) International application number:
**PCT/JP2006/313499**

(87) International publication number:
**WO 2007/102230 (13.09.2007 Gazette 2007/37)**

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **07.03.2006 PCT/JP2006/304370**

(71) Applicant: **Osaka University
Suita-shi
Osaka 565-0871 (JP)**

(72) Inventors:
• **UCHIYAMA, Susumu
Suita-shi
Osaka 565-0871 (JP)**
• **FUKUI, Kiichi
Suita-shi
Osaka 565-0871 (JP)**

(74) Representative: **Müller, Christian Stefan Gerd et al
Dr. Volker Vossius
Patent- und Rechtsanwaltskanzlei
Geibelstrasse 6
81679 München (DE)**

(54) **HUMANIZED ANTI-CD20 MONOCLONAL ANTIBODY**

(57)    The present invention provides a humanized anti human CD20 monoclonal antibody, selection criteria therefor, humanized antibodies selected using that criteria and showing biological characteristics suitable for use as pharmaceuticals.

**Description**

TECHNICAL FIELD

The present invention relates to an anti CD20 monoclonal antibody.

BACKGROUND ART

CD20 is a protein not containing sugar chains, which is expressed on the cellular surface of human B lymphocytes. CD20 is expressed in the B cells of many malignant tumors in addition to expression in normal B cells in the peripheral blood, spleen, tonsils and bone marrow.

Epitopes to which monoclonal antibodies of CD20 bind display extremely high variation and a wide variety of biological responses have been reported. Furthermore there have been many reports of monoclonal antibodies recognizing CD20. In particular, rituximab is a chimerized murine/human monoclonal antibody (C2B8) derived from a murine antibody 2B8 obtained by immunizing an SB cell strain, a type of human B cell (refer to Pamphlet of WO 94/11026; and US Patent No. 5,736,137). Rituximab is used under the name Rituxan® as a therapeutic agent for the treatment of low malignant non-Hodgkin's lymphoma (NHL). Subsequently it was reported that Rituxan is effective for many immune diseases related with B cells. For example, Rituxan has demonstrated efficacy against malignant tumors such as chronic lymphocytic leukemia (CCL), autoimmune diseases involving pathogenic autoantibodies such as autoimmune hemolyticanemia and idiopathic thrombocytopenic purpura (ITP), and inflammatory diseases such as rheumatoid arthritis (RA) and multiple sclerosis (refer to Coiffier B et al., Blood 1998; 92:1297-32; Edward JC et al., Rheumatology (Oxford) 2001; 40:205-11; Zaja F et al., Heamatologica 2002; 87:189-95; and Perrotta S et al., Br J Haematol 2002; 116:465-7).

It has been reported that human complement binds to rituximab conjugated to lymphatic B cells resulting in lysis of lymphatic B cell lines by complement-dependent cytotoxicity (CDC) (refer to Reff et al., Blood 1994; 83: 435-445). Rituximab has also displayed activity in assays for antibody-dependent cell-mediated cytotoxicity (ADCC) and induced apoptosis and growth inhibitory activity in tritiated thymidine incorporation assays (refer to Maloney et al., Blood 1996; 88: 637a).

Molecules chimerized from different animal types are antigenic and therefore are generally not desirable as therapeutic agents. However anti CD20 antibodies including rituximab are not antigenic since they target all B cells including normal cells followed by their deletion. However it has been reported that neutralizing antibodies, albeit several percent, are induced during therapy and that the level of dosage and therapeutic periods increase the probability of inducing neutralizing antibodies. In addition, there has been a shift in treatment targets from B cell lymphomas to RA, IT and MS. Therefore there is an increased focus on problems associated with antigenicity. Consequently recently there has been a need for human antibodies or humanized antibodies containing sequences close to human sequences.

Chimerized antibodies entail the problem that they have a relatively short half life in blood. The $\beta$ half life ($\beta$ 1/2) of murine/human chimerized antibodies including rituximab is no more than 3 to 4 days. The efficacy in clinical trials of rituximab against low malignant NHL has been reported to be less than 50% (refer to IDEC Pharmaceuticals Corporation News Release, December 8, 1998). Furthermore there is the problem that increases in dosages required in NHL therapy since the dissociation constant (Kd value) of rituximab for CD20 antibody is 5.2 nM and the corresponding binding affinity is not very high (refer to Mitchell ER et al., Blood 1994; 82:435-445).

DISCLOSURE OF THE INVENTION

In view of the problems discussed above, the present invention has the principal object of providing an anti CD20 monoclonal antibody displaying biological activity suitable as a pharmaceutical.

In order to attain the above object, the present inventors performed diligent research into the preparation of a monoclonal antibody displaying high binding affinity to naturally-occurring human CD20 molecules in order to obtain an anti CD20 monoclonal antibody having excellent characteristics. As a result, the invention is based on the insight that a high-affinity monoclonal antibody displaying excellent biological activity is obtained by use of an immunogen such as SB cells or Raji cells which are B cell strains thought to contain a high density of CD20 antigen combined with a non-human animal cell modified using genetic recombination to express large amount of CD20 on the cellular membrane.

The present inventors succeeded in identifying a novel method of selecting effective anti human CD20 humanized antibodies. The use of this method of selection has enabled the selection of candidates from the humanized anti CD20 monoclonal antibodies of the present invention for use as effective therapeutic agents.

In other words, the present invention provides the followings:

(1) A humanized anti CD20 monoclonal antibody which has growth inhibiting activity on cells having human CD20 antigen, the immunogen being a human B cell strain expressing human CD20 antigen and a cell strain transformed with human CD20 DNA, which is a non-human cell and derived from an animal which is different from the animal to be immunized, and which meets the selection criteria below:

(i) the antibody having a dissociation constant (Kd value) for human CD20 antigen of less than approximately 9.5 nM and CDC activity on B cells equal to or greater than that of 2B8 antibody;

(2) A humanized anti CD20 monoclonal antibody which has growth inhibiting activity on cells containing human CD20 antigen, the immunogen being a human B cell strain expressing human CD20 antigen and a cell strain transformed with human CD20 DNA, which is a non-human cell and derived from an animal which is different from the animal to be immunized, and which meets the selection criteria below:

(a) the antibody having a dissociation constant (Kd value) for human CD20 antigen of less than approximately 9.5 nM and CDC activity on Raji cells (suspended cells) or SU-DHL4 cells equal to or greater than that of 2B8 antibody;

(3) A humanized anti CD20 monoclonal antibody which has growth inhibiting activity on cells containing human CD20 antigen, the immunogen being a human B cell strain expressing human CD20 antigen and a cell strain transformed with human CD20 DNA, which is a non-human cell and derived from an animal which is different from the animal to be immunized, and which meets the selection criteria below:

(ii) the antibody having a Kd value for human CD20 antigen in the range of from approximately 9.5 nM to approximately 13 nM and a total of apoptosis activity and CDC activity on B cells equal to or greater than that of 2B8 antibody;

(4) A humanized anti CD20 monoclonal antibody which has growth inhibiting activity on cells containing human CD20 antigen, the immunogen being a human B cell strain expressing human CD20 antigen and a cell strain transformed with human CD20 DNA which is a non-human cell and derived from an animal which is different from the animal to be immunized, and which meets the selection criteria below:

(b) the antibody having a Kd value for human CD20 antigen in the range of from approximately 9.5 nM to approximately 13 nM and a total of apoptosis activity and CDC activity on WiL2 cells or RCK8 cells equal to or greater than that of 2B8 antibody;

(5) The humanized anti CD20 monoclonal antibody according to the above-described 1 or 2, comprising a combination of the L chain set forth in SEQ ID No: 18 and the H chain set forth in SEQ ID No: 22;
(6) The humanized anti CD20 monoclonal antibody according to the above-described (1) or (2), comprising a combination of the L chain set forth in SEQ ID No: 18 and the H chain set forth in SEQ ID No: 24;
(7) The humanized anti CD20 monoclonal antibody according to the above-described (3) or (4), comprising a combination of the L chain set forth in SEQ ID No: 19 and the H chain set forth in SEQ ID No: 22; and
(8) A therapeutic agent for the treatment of B cell mediated diseases, comprising as an active ingredient the humanized anti CD20 monoclonal antibody according to any one of the above-described (1) to (7).

The present invention provides a humanized anti CD20 monoclonal antibody displaying a high binding affinity against extracellular epitopes of CD20 antigen and high cytotoxic activity such as CDC. These antibodies are extremely effective as therapeutic agents for diseases related with B cells.

BRIEF DESCRIPTION OF THE DRAWINGS

Fig. 1 is a restriction map showing the structure of a vector pNOW-Ab for expressing a transformed antibody.
Fig. 2 is a restriction map showing the structure of a vector pNOW for expressing protein.
Fig. 3a is a graph showing the results of an apoptosis test.
Fig. 3b is a graph showing the results of an apoptosis test.
Fig. 3c is a graph showing the results of an apoptosis test.
Fig. 3d is a graph showing the results of an apoptosis test.
Fig. 4a is a graph showing the relationship of antibody concentration and ADCC.
Fig. 4b is a graph showing the relationship of antibody concentration and ADCC.

Fig. 4c is a graph showing the relationship of antibody concentration and ADCC.

Fig. 4d is a graph showing the relationship of antibody concentration and ADCC.

Fig. 5a is a graph showing the relationship of E:T ratio and ADCC.

Fig. 5b is a graph showing the relationship of E:T ratio and ADCC.

Fig. 5c is a graph showing the relationship of E:T ratio and ADCC.

Fig. 5d is a graph showing the relationship of E:T ratio and ADCC.

Fig. 6a is a graph showing the results of a CDC test.

Fig. 6b is a graph showing the results of a CDC test.

Fig. 6c is a graph showing the results of a CDC test.

Fig. 6d is a graph showing the results of a CDC test.

Fig. 7a is a graph showing the results of an apoptosis test using a murine antibody.

Fig. 7b is a graph showing the results of an apoptosis test using a murine antibody.

Fig. 7c is a graph showing the results of an apoptosis test using a murine antibody.

Fig. 7d is a graph showing the results of an apoptosis test using a murine antibody.

Fig. 8a is a graph showing the results of an apoptosis test using a humanized antibody.

Fig. 8b is a graph showing the results of an apoptosis test using a humanized antibody.

Fig. 8c is a graph showing the results of an apoptosis test using a humanized antibody.

Fig. 8d is a graph showing the results of an apoptosis test using a humanized antibody.

Fig. 9a is a graph showing the ratio of early apoptosis test using a humanized antibody.

Fig. 9b is a graph showing the ratio of early apoptosis test using a humanized antibody.

Fig. 9c is a graph showing the ratio of early apoptosis test using a humanized antibody.

Fig. 9d is a graph showing the ratio of early apoptosis test using a humanized antibody.

Fig. 10a is a graph showing the relationship between dissociation constant of humanized antibody and cytotoxicity (apoptosis inducing activity and CDC activity). Cells used are Raji cells. The white square shows CDC activity (%), the black circle shows apoptosis activity (%).

Fig. 10b is a graph showing the relationship between dissociation constant of humanized antibody and cytotoxicity (apoptosis inducing activity and CDC activity). Cells used are SU-DHL4 cells. The white square shows CDC activity (%), the black circle shows apoptosis activity (%).

Fig. 10c is a graph showing the relationship between dissociation constant of humanized antibody and cytotoxicity (apoptosis inducing activity and CDC activity). Cells used are WiL2 cells. The white square shows CDC activity (%), the black circle shows apoptosis activity (%).

Fig. 10d is a graph showing the relationship between dissociation constant of humanized antibody and cytotoxicity (apoptosis inducing activity and CDC activity). Cells used are RC-K8 cells. The white square shows CDC activity (%), the black circle shows apoptosis activity (%).

Fig. 11a is a graph showing CDC activity of humanized antibody and chimerized antibody. Cells used are Raji cells.

Fig. 11b is a graph showing CDC activity of humanized antibody and chimerized antibody. Cells used are SU-DHL4 cells.

Fig. 11c is a graph showing CDC activity of humanized antibody and chimerized antibody. Cells used are WiL2 cells.

Fig. 11d is a graph showing CDC activity of humanized antibody and chimerized antibody. Cells used are RC-K8 cells.

Fig. 12a is a graph showing the relationship of humanized antibody concentration and ADCC. Cells used are Raji cells.

Fig. 12b is a graph showing the relationship of humanized antibody concentration and ADCC. Cells used are SU-DHL4 cells.

Fig. 12c is a graph showing the relationship of humanized antibody concentration and ADCC. Cells used are WiL2 cells.

Fig. 12d is a graph showing the relationship of humanized antibody concentration and ADCC. Cells used are RC-K8 cells.

Fig. 13a is a graph showing the relationship of the E:T ratio for humanized antibody and ADCC. Cells used are Raji cells.

Fig. 13b is a graph showing the relationship of the E:T ratio for humanized antibody and ADCC. Cells used are SU-DHL4 cells.

Fig. 13c is a graph showing the relationship of the E:T ratio for humanized antibody and ADCC. Cells used are WiL2 cells.

Fig. 13d is a graph showing the relationship of the E:T ratio for humanized antibody and ADCC. Cells used are RC-K8 cells.

Description of Abbreviations:

Pcmv: Cytomegalovirus promoter

PABgh: Signal with poly A for bovine growth hormone gene
Psvd: Promoter for simian virus 40 deficient in enhancer
DHFR: cDNA for murine dihydrofolic acid reductase
PAsv: Signal with poly A for simian virus 40
PBR322ori: Replication origin in E. coli
Amp[r]: Selective marker in E. coli (ampicillin resistance)
Neo[r]: Selective marker in mammalian cells (G418 resistance)
INrbg: Intron for rabbit $\beta$ globin
SP1: Signal peptide for antibody light chain
VL: cDNA for antibody light chain variable region
C$\kappa$: cDNA for antibody $\kappa$ light chain constant region
SPh: Signal peptide for antibody light chain
Vh: cDNA for antibody light chain variable region
Cy1: cDNA for antibody $\gamma$1 heavy chain constant region

BEST MODE FOR CARRYING OUT THE INVENTION

In this specification, "antibody" refers to not only a whole antibody but also a fragment displaying equivalent binding affinity to antigen as that of the whole antibody, and for example, includes fragments (Fab, F(ab')$_2$) containing the variable region of the original whole antibody.
The monoclonal antibodies according to the present invention are monoclonal antibodies which display high affinity to human CD20 antigen and have excellent biological activity and include murine-derived monoclonal antibodies, in addition to chimerized and humanized variants thereof.
A monoclonal antibody according to a first preferred embodiment has growth inhibiting activity on cells expressing human CD20 antigen, has a dissociation constant (Kd value) for human CD20 antigen of less than 1/2 of 2B8, which is a murine antibody derived from Rituximab, and preferably a Kd value of 1.7 to 3.39 nM and a high affinity to human CD20 antigen. There is no particular limitation on the method of measuring the dissociation constant (Kd value) as long as the method allows the measurement of a Kd value with respect to antigen presented on cells. However in this specification, the dissociation constant is taken to be obtained by the method described hereinafter in the

Examples.

The growth inhibitory activity against cells expressing human CD20 antigen should preferably be greater than that of 2B8. The growth inhibitory activity is preferably growth inhibitory activity with respect to in vitro culturing of cells expressing human CD20 antigen cultured in the absence of peripheral blood monocytes. More preferably, the growth inhibitory activity induces apoptosis. It has been reported that the binding of some anti CD20 antibodies to CD20 increases the concentration of calcium ions (Ca$^{2+}$) in B cells and thus induces apoptosis mediated by Src kinase.
The measurement of the growth inhibitory activity above may be performed using the method described in Miyamoto T, Min W, Lillehoj HS.Avian Dis. 2002 Jan-Mar; 46(1):10-6.
A specific example of a monoclonal antibody according to a first embodiment of the present invention is a murine-derived monoclonal antibody wherein the L chain variable region amino acid sequence and the H chain variable region amino acid sequence are respectively SEQ ID Nos: 1 and 9, SEQ ID Nos: 2 and 10, or SEQ ID Nos: 3 and 11 in addition to chimerized or humanized variants thereof.
A chimerized antibody may be produced by fusing a variable region amino acid sequence from a murine-derived monoclonal antibody with a human immunoglobin constant region amino acid sequence according to a known method such as described for example in Ishida T, Imai K, Nippon Rinsho Vol 60, No 3, 2002-3:439-444. Humanizing may be performed for example using a variable region CDR amino acid sequence from a murine-derived monoclonal antibody and a human immunoglobin amino acid sequence according to a known method such as described in Ishida T, Imai K, Nippon Rinsho Vol 60, No 3, 2002-3:439-444, Eduardo A.Padlan, Molecular Immunology, Vol. 28-4/5, pp489-498, 1991; Eduardo A. Padlan et.al., The FASEB Journal,vol.9, pp133-139; and Tai te Wu, Elvin A. Kabat, Molecular Immunology, Vol.29-9, pp1141-1146, 1992.
When producing chimerized or humanized antibodies, an amino acid sequence from an L chain variable region of a plurality of murine monoclonal antibodies may be combined in an arbitrary manner with amino acid sequences from the H chain variable region. Examples include a chimerized anti CD20 monoclonal antibody combining a chimerized H chain of a murine-derived monoclonal antibody variable region amino acid sequence set forth in SEQ ID Nos: 9 to 11 and a chimerized L chain of a murine-derived monoclonal antibody variable region amino acid sequence set forth in any one of SEQ ID Nos: 1 to 3 and a humanized anti CD20 monoclonal antibody combining a humanized H chain of a murine-derived monoclonal antibody variable region CDR sequence set forth in any one of SEQ ID Nos: 9 to 11 and a humanized L chain of a murine-derived monoclonal antibody variable region CDR amino acid sequence set forth in any one of SEQ ID Nos: 1 to 3.

An antibody according to a second preferred embodiment of the present invention is a murine-derived chimerized or humanized monoclonal antibody which has a dissociation constant (Kd value) less than or equal to 1/8 that of 2B8 for human CD20 antigen.

It is well known that when an antibody which has a high affinity for human CD20 antigen, and in particular an antibody containing human IgG1 or IgG3 or an engineered a human Fc sequence aversion thereof (including murine-derived chimerized or humanized monoclonal antibodies) binds to CD20 on the cellular membrane, effector-cell activity is induced via FcγRIII (CD16) on NK cells resulting in antibody-dependent cell-mediated cytotoxicity (ADCC). It is well known that when an antibody which has high affinity to humanized CD20 antigen, and in particular an antibody containing human IgG1 or IgG3 or an engineered a human Fc sequence version thereof (including murine-derived chimerized or humanized monoclonal antibodies) binds to CD20 on the cellular membrane, the antibody induces complement activity and causes complement-dependent cytotoxicity (CDC).

Therefore an antibody according to a second preferred embodiment of the present invention is expected to display ADCC or CDC.

A specific example of an antibody according to a second embodiment of the present invention an antibody wherein the L chain variable region amino acid sequence and the H chain variable region amino acid sequence are respectively SEQ ID Nos: 4 and 12, SEQ ID Nos: 5 and 13, SEQ ID Nos: 6 and 14, SEQ ID Nos: 7 and 15 or SEQ ID Nos: 8 and 16. Chimerized or humanized antibodies can be produced by a similar method to the first embodiment. An amino acid sequence from an L chain variable region of a plurality of murine-derived monoclonal antibodies may be combined in an arbitrary manner with amino acid sequences from the H chain variable region. Examples include a chimerized anti CD20 monoclonal antibody combining a chimerized H chain of a murine-derived monoclonal antibody variable region amino acid sequence set forth in any one of SEQ ID Nos: 12 to 16 and a chimerized L chain of a murine-derived monoclonal antibody variable region amino acid sequence set forth in any one of SEQ ID Nos: 4 to 8, and a humanized anti CD20 monoclonal antibody combining a humanized H chain of a murine-derived monoclonal antibody variable region CDR sequence set forth in any one of SEQ ID Nos: 12 to 16 and a humanized L chain of a murine-derived monoclonal antibody variable region CDR amino acid sequence set forth in any one of SEQ ID Nos: 4 to 8.

An antibody according to a third preferred embodiment of the present invention is a group of humanized monoclonal antibody not limited by a specific dissociation constant (Kd value) with respect to 2B8 and includes humanized monoclonal antibodies effective against cells with respect to which rituximab is not effective.

Examples of such antibodies include a humanized anti CD20 monoclonal antibody combining an L chain set forth in SEQ ID No: 18 and an H chain set forth in SEQ ID No: 24, an L chain set forth in SEQ ID No: 18 and an H chain set forth in SEQ ID No: 22, an L chain set forth in SEQ ID No: 19 and an H chain set forth in SEQ ID No: 22 and an L chain set forth in SEQ ID No: 19 and an H chain set forth in SEQ ID No: 23.

Furthermore the present inventors have classified humanized anti CD20 monoclonal antibodies obtained by the present invention by type based on a relationship between antibody affinity to human CD20 antigen and CDC activity, and apoptosis inducing activity and have selected monoclonal antibodies for use as antibody pharmaceuticals based on that classification (refer to Example 4).

In other words, the inventors realized that high-affinity antibodies could not induce apoptosis independently and required cross linking by a secondary antibody in order to induce apoptosis. On the other hand, the inventors realized that low affinity antibodies could induce apoptosis in isolation. Furthermore high antibody affinity was often found to correlate with high CDC activity. As a result, although low affinity antibodies could induce apoptosis without the presence of a secondary antibody, there was a tendency for low CDC activity. Thus these observations resulted in the formation of two selection criteria for candidate antibodies effective as therapeutic agents:

An antibody which can not induce apoptosis independently but which displays extremely high affinity to human CD20 antigen and CDC-mediated anti-cancer effects, or
An antibody which can induce apoptosis independently and which has high affinity to human CD20 antigen in addition to being an antibody which displays anti-cancer effects due to apoptosis and CDC.

In the former selection criterion, it is preferred that antibodies are selected which have a high affinity to human CD20 antigen and which have high CDC activity (display an inverse correlation between Kd value and CDC activity). Although clones satisfying the former selection criterion do not induce apoptosis independently, such clones have the advantage of high affinity and thus it is possible to promote cell deletion by CDC activity. The present inventors made the surprising discovery that a majority of antibodies satisfying the former selection criterion have a dissociation constant (Kd value) for human CD20 antigen of less than approximately 9.5 nM.

In the latter selection criterion, it is preferred that antibodies are selected which have a large total of CDC and apoptosis activity and which have a high affinity to human CD20 antigen. Although clones satisfying the latter selection criterion do not display high affinity, such clones have the advantage of high apoptosis activity and thus it is possible to promote cell deletion by a synergistic effect of CDC and inducing apoptosis. The present inventors made the surprising discovery that a majority of antibodies satisfying the latter selection criterion have a dissociation constant (Kd value) for human CD20 antigen in the range from approximately 9.5 nM to approximately 13 nM.

It is preferred that the selection criteria are expressed numerically. Thus it is possible to express the selection criteria in as follows:

(i) An antibody having a dissociation constant (Kd value) for human CD20 antigen of less than approximately 9.5 nM and CDC activity against B cells of equal to or greater than 2B8 antibody; or
(ii) An antibody having a Kd value for human CD20 antigen in the range of from approximately 9.5 nM to approximately 13 nM and a total of CDC activity and apoptosis activity against B cells equal to or greater than 2B8 antibody.
Herein, "equal" refers to a value in a range of approximately ±10% of the figure under comparison.

Experiments performed by the present inventors lead to the surprising result that it is preferred to apply the selection criteria according to the type of B cell. More precisely, in view of the results obtained in Example 4 described hereinafter, it is possible to define selection criterion (i) in greater detail in (a), and selection criteria (ii) in (b).

(a) An antibody having a dissociation constant (Kd value) for human CD20 antigen of less than approximately 9.5 nM and CDC activity against Raji cells (floating cells) or SU-DHL4 cells of equal to or greater than 2B8 antibody; or
(b) An antibody having a Kd value for human CD20 antigen in the range of from approximately 9.5 nM to approximately 13 nM and a total of CDC activity and apoptosis activity against WiL2 cells or RCK8 cells equal to or greater than 2B8 antibody.
Herein, "equal" refers to a value in a range of approximately ±10% of the figure under comparison.

When applying the selection criteria in (i) or (a) above, it is preferred to select antibodies which have a Kd value for human CD20 antigen of less than approximately 9.5 nM, which is as low a Kd value as possible, and which have high CDC activity. Preferably, antibodies are selected which have CDC activity against Raji cells (floating cells) or DHL4 cells which is equal to or greater than 2B8 antibody. Normally there is a tendency that antibody with lower Kd values has higher antibody CDC activity, it is possible to simply select antibodies with small Kd values (high affinity to human CD20 antigen). Since antibodies satisfying selection criteria (i) or (a) have extremely high affinity to CD20 antigen as well as extremely high CDC activity, such antibodies display excellent anti-cancer effects. However antibodies satisfying selection criteria (i) or (a) can not induce apoptosis independently and require a secondary antibody in order to induce apoptosis.

When applying the selection criteria in (ii) or (b) above, it is preferred to select antibodies which have a small Kd value for human CD20 antigen in the range of approximately 9.5 nM to approximately 13 nM and which display the highest possible total of apoptosis activity and CDC activity. Preferably, antibodies are selected which have a total of apoptosis activity and CDC activity against WiL2 cells or RCK8 cells and the total is equal to or greater than 2B8 antibody. Since antibodies satisfying selection criteria (ii) or (b) have moderately high affinity to CD20 antigen and can induce apoptosis independently, such antibodies can display excellent anti-cancer effects as a result of the synergistic effect between apoptosis and CDC. Preferably, antibodies are selected which have a total of CDC activity and apoptosis activity equal to or greater than 2B8 antibody.

In other words, selection criteria (i) or (a) can be applied when it is expected that the antibody will have extremely high CDC activity. Selection criteria (ii) or (b) can be applied when it is expected that the antibody will have both CDC activity and apoptosis activity.

An example of a humanized CD20 monoclonal antibody obtained by the present invention which satisfies selection criteria (i) or (a) is a humanized CD20 monoclonal antibody combining an L chain set forth in SEQ ID No: 18 and an H chain set forth in SEQ ID No: 22 and a humanized CD20 monoclonal antibody combining an L chain set forth in SEQ ID No: 18 and an H chain set forth in SEQ ID No: 24.
An example of a humanized CD20 monoclonal antibody obtained by the present invention which satisfies selection criteria (ii) or (b) is a humanized CD20 monoclonal antibody combining an L chain set forth in SEQ ID No: 19 and an H chain set forth in SEQ ID No: 22.

It is possible to apply the selection process and methodology related to selection according to the present invention to antibodies recognizing epitopes which are different from the antibodies of the present invention, for example, antibodies obtained by a method other than that of the present invention. Thus the invention in a further embodiment relates to a method of selection of anticancer antibodies and antibodies selected by use of the method, the antibodies characterized by the following selection criteria:

An antibody which can not induce apoptosis independently but which displays extremely high affinity to antigen and CDC-mediated anti-cancer effects, or

An antibody which can induce apoptosis independently and which has high affinity to antigen in addition to being an antibody which displays anti-cancer effects due to apoptosis and CDC.

When applying selection criteria (i) or (a), or (ii) or (b), the measurement of affinity to antigen, CDC activity or apoptosis activity may be performed using any known method in the relevant field. Measurement of affinity to antigen is generally performed by using the measured the dissociation constant with respect to antigen as a standard. Measurement of the dissociation constant of humanized antibody with respect to human CD20 antibody is generally performed using cells expressing human CD20 antigen as a standard. It is preferred to use cells not expressing human CD20 antigen as a control. Methods such as attaching a detectable label to the humanized antibody, or using a labeled antibody specific to a human antibody can be used to detect humanized antibody binding to cells. For example, measurement of affinity to CD20 antigen (or dissociation constant, Kd values) can be performed as described in Example 2 hereinafter.

Isolation and selection of humanized anti CD20 antibodies according to the present invention will be described hereinafter.

A murine-derived monoclonal antibody which can be used in the preparation of a humanized anti CD20 antibody according to the present invention can be prepared by selecting a clone producing a monoclonal antibody having target characteristics from hybridoma clones produced by screening with the methods described hereinafter.

Sensitizing antigen (immunogen) can be obtained from SB cells or Raji cells which are cells expressing CD20 and, for example, CHO cells (CHO/CD20) expressing CD20 on the cellular membrane transformed by recombinant techniques using commercially available CD20 DNA (or filaments having the same effect). During initial immunization, additional immunization(s) and final immunization, immunization should be performed at least once during initial immunization and additional immunization(s) using either a cell strain which presents the sensitizing antigen and is derived from an animal of a different order from the animal being immunized or using a cell strain which presents the sensitizing antigen on the cellular surface membrane as a result of genetic recombination and is derived from an animal of the same order as the animal being immunized. The other cell strain is used during final immunization.

Other conditions may be the same as normal conditions for methods of preparing hybridomas producing monoclonal antibodies. Hybridomas producing monoclonal antibodies are prepared by known methods such as (1) immunizing an animals to be immunized (2) preparation of lymphocytes from immunized animals, (3) preparation of parent cells, (4) cell fusion of lymphocytes and parent cells and (5) screening and cloning (see Monokuronaru kotai, Seikagaku Jikkenho (Monoclonal Antibody, Biochemical Experiment Method), edited by Ailsa M. Campbell, and translated by Toshiaki OSAWA, Tokyo Kagaku Dojin (1989).

Methods of preparing monoclonal antibodies using cloned hybridomas may employ hybridomas prepared using the method of hybridoma production according to the present invention or may use a widely employed method of preparing monoclonal antibodies. Large-scale production can be effected for example by methods of cell culture or methods of producing murine ascites. Production of chimerized or humanized antibodies can be performed by producing genes coding for the chimerized or humanized antibody, transforming an expression vectors with the genes and expressing the expression vector in a suitable cell.

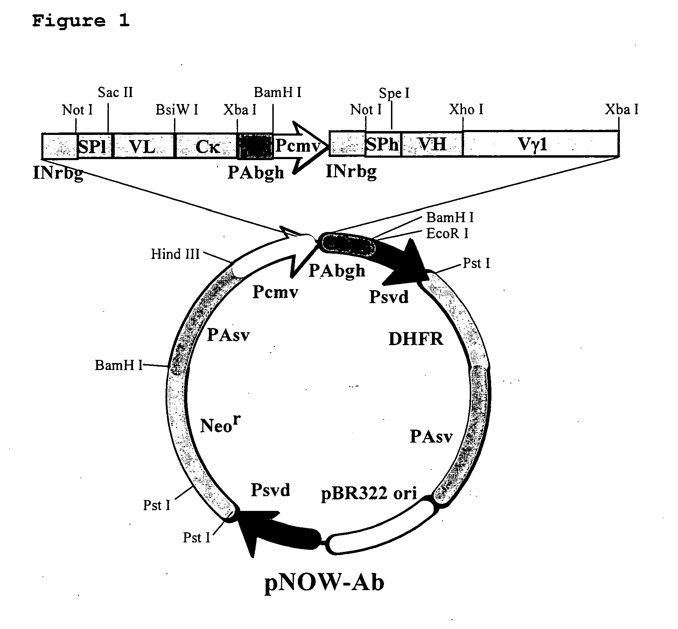

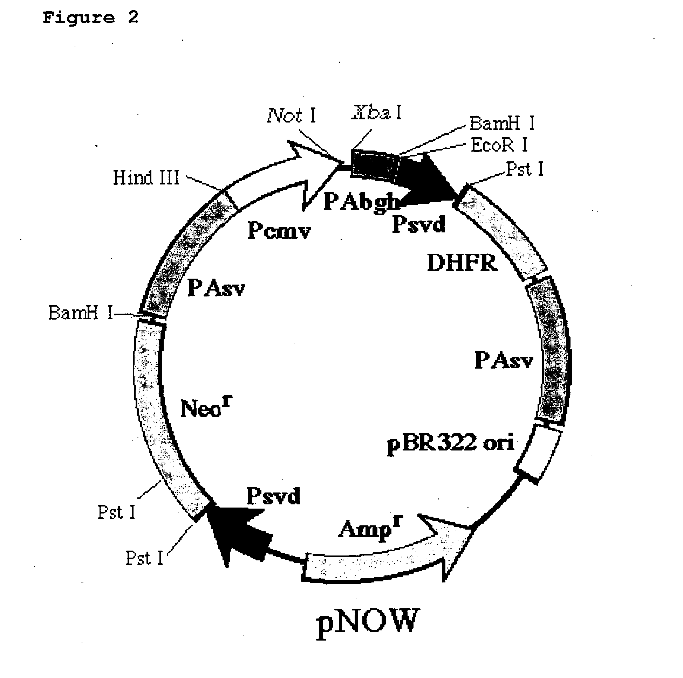

For example, variable region genes for the L chain and the H chain can be chimerized using constant regions genes for human immunoglobin L chain and H chain ($\kappa$) and combined with a CHO cell high expression vector. Although a commercially available vector system for production of recombinant antibodies can be used, it is possible to use a dimmer high expression vector pNOW-ab containing a multicloning site (MCS) for both L chains and H chains which is based on a high expression vector for mammalian cells (Japanese Patent No. 3,582,965). Restriction maps showing the structure of the vectors are shown in Figs. 1 and 2. The expression vectors containing chimerized antibody genes are used to transfect CHO cells and then highly-productive clones are isolated. Antibodies are produced from these clones using known methods.

An antibody according to a first embodiment of the present invention displays relative high binding affinity compared to rituximab and growth inhibition activity. Preferably, since the antibody displays high activity in inducing apoptosis, a chimerized or humanized antibody can be used as an active ingredient of a therapeutic agent against diseases involving B cells and against B cell malignant tumors. Furthermore antibodies according to the second and third embodiments of the present invention are thought to display complement-dependent cytotoxicity (CDC) and antibody-dependent cell-mediated cytotoxicity (ADCC) against cells expressing human CD20 antigen and thus can be used as an active ingredient of a therapeutic agent against immune diseases involving B cells and against B cell malignant tumors. Thus the present invention also provides a therapeutic agent against diseases involving B cells which has as an active ingredient the chimerized or humanized antibodies of the invention.

The humanized anti CD20 monoclonal antibody according to the present invention can be selected using the selection criteria in (i) or (a), or in (ii) or (b) above. Antibodies satisfying these criteria are highly effective against immune diseases involving B cells and B cell malignant tumors and are particularly suitable for pharmaceutical use. Thus the invention provides a therapeutic agent for B cell mediated diseases containing, as an active ingredient, a humanized anti CD20 monoclonal antibody satisfying selection conditions (i) or (a), or (ii) or (b) above.

Two or more types of antibodies according to the present invention can be combined.

Diseases involving B cells are not limited to the following and include for example non-Hodgkin's lymphoma, chronic lymphocytic leukemia, acute lymphocytic leukemia, rheumatoid arthritis, autoimmune hemolyticanemia, idiopathic thrombocytopenic purpura, systemic lupus erythematosus, anti-phospholipid antibody syndrome, xerodermosteosis, Crohn's disease, chorionitis, and multiple sclerosis.

The therapeutic agent can be produced by known techniques and there is not particular limitation on processing ingredients. Dosages and the like can be determined by reference to known dosages for Rituxan.

The present invention will be described in further detail hereinafter with reference to the Examples. However the invention is not limited to the Examples.

EXAMPLE 1

(1) Preparation of Immunogen for Immunosensitizing Mice

SB cells and Raji cells which are B cells strains expressing CD10 were cultured *in vitro*.

Separately thereto, DNA coding for the entire CD20 molecule (Multiple Choice cDNA human spleen, Origene Technologies, Inc. 6 Taft Court, Suite 100, Rockville, MD 20850) was cloned using specific primers hCD20-S-GK-Not aatgcggccgccaccatgacaacacccagaaattc (SEQ ID No: 25) and hCD20-E-Xba gctctagattaaggagagctgtcattttc (SEQ ID No: 26). The DNA was inserted into a pNOW high expression vector for mammalian cells (Fig. 1) and used to transform CHO cells. Recombinant CHO cells (CD20/CHO cells) displaying high level of CD20 expression on the cellular surface were identified using FACS analysis. Staining was performed using FITC-labeled CD20 monoclonal antibodies and cells were selected as high expression cells when expressing five times or more the fluorescent intensity of SB cells.

(2) Preparation of Immunogen

SB cells and Raji cells were cultured using RPM1640 medium supplemented with 10% FCS. CD20/CHO cells were cultured using CHO-S-SFMII medium (Gibco, Cat. No. 12052-098) supplemented with 800 $\mu$g/ml of G418. These culture mediums were centrifuged for 5 minutes at 1,100 rpm, the cells were suspended in Dulbecco's PBS(-) and centrifuged again. This washing step was performed again, physiological salt solution was added to the cells and the prepared suspension (number of cells: 1-3 x $10^7$/ml) used for immunization.

(3) Immunization

Both immunogen preparations were administered intraperitoneally to a 7-11 week female Balb/c mouse. After administering either SB cells or CD20/CHO cells on 2 - 3 occasions at various daily intervals, final immunization was performed using another cell type (CD20/CHO cells or Raji cells). The number of cells administered was 1-3 x $10^7$ cells per mouse for any type of cell.

The combinations of immunogens are shown in Table 1.

(4) Cell Fusion

Three days after final immunization, spleen cells were recovered from two mice and fused with murine myeloma (NS-1) in the presence of PEG-1500 using the method described in Oi, V.T. and L.A. Herzenberg, 1980, in: Selected Methods in Cellular Immunology, eds. B. Mishell and S.M. Shiigi (Freeman and Co. San Francisco, CA) p. 351.

(5) Primary and Secondary Screening

A Cell ELISA assay was performed using a 96-wellplate having CD20/CHO cells or CHO cells (parent strain) attached thereto and wells producing antibodies reacting specifically to CD20 were selected. The same 96-wellplate with CD20/CHO cells attached thereto was used to perform a competitive reaction with rituximab (C2B8). Antibodies (wells) were selected which reacted to epitopes similar to C2B8.

The results of the screening are shown in Table 1.

(6) Cell ELISA

CD20/CHO cells or CHO cells (parent strain) attached to a Poly-L-Lysine coated 96-wellplate (Asahi Techoglass Corporation, Cat. No. 11-023-018) were used in a cell ELISA assay. 150 $\mu$l of blocking buffer (PBS solution with 0.2% gelatin, 0.5% BSA) was introduced into each well and the plate was allowed to stand at 37°C for one hour. The plate was washed five times using an aqueous solution of 150 nM-NaCl, 0.05%-Tween20 and then a 100 μl sample (a diluted solution of the culture supernatant) was introduced into each well. The primary reaction was conducted at 37°C for one hour. After washing, 100 μl of a diluted solution of a labeled antibody (HRP-labeled anti murine IgG (H+L) rabbit antibody (Jackson Lab. Code No. 315-035-003) or HRP-labeled anti murine IgG (Fcγ) rabbit antibody (Jackson Lab. Code No. 315-035-008)) was introduced into each well and a secondary reaction was conducted at 37°C for one hour. The same blocking solution was used in the preparation of the reaction solution for the primary and the secondary reactions. After washing, 100 μl of a color development solution (OPD) was introduced into each well and after 30 minutes 50 μl of 4N-$H_2SO_4$ was added to stop the reaction. The absorbance was measured at 492 nm.

(7) Competitive Reaction in Cell ELISA

A mixed solution of a sample (diluted solution of culture supernatant) and chimerized antibody (10 to 40 ng/ml) was prepared.

After performing a blocking reaction as described above with respect to the Cell ELISA assay, 100 μl of the mixed solution was introduced into each well and the primary reaction was performed at 37°C for one hour. After washing, 100 μl of a diluted solution of a labeled antibody (HRP-labeled anti human IgG (H+L) rabbit antibody (Jackson Lab. Code No. 309-035-082)) was introduced into each well and a secondary reaction was conducted at 37°C for one hour. After washing, 100 μl of a color development solution (OPD) was introduced into each well and after 30 minutes 50 μl of 4N-$H_2SO_4$ was added to stop the reaction. The absorbance was measured at 492 nm.

Since the labeled antibody only reacts with the chimerized antibody, a reduction in the measured value should result from competition between the antibodies in the sample added in the primary reaction and the chimerized antibodies.

(8) Cloning

A limiting dilution method was used. After culturing cells dispersed on a 96 wellplate, a Cell ELISA assay was performed on the culture supernatant of a well having a single colony in order to select clones producing specific antibodies.

(9) Preparation of Purified Antibody

Clones producing specific antibodies were cultured in RPMI1640 medium supplemented with 10% FCS. When the cell density reached a value of approximately $5 \times 10^5$/ml, the medium was replaced by serum-free culture medium ASF-104N (Ajinomoto). After 2 to 4 days, the culture solution was centrifuged, the culture supernatant recovered and a protein G column used to purify the antibody. The monoclonal antibody elution was dialyzed using 150 mM - NaCl. Filtration sterility was performed using a 0..2 μm filter in order to obtain the test antibody (anti human CD20 murine monoclonal antibody).

Table 1

| Cell Fusion Series | Immunizing Method | | | Primary, Secondary Screening Specificity against CD20 on CD20/CHO cells | | |
|---|---|---|---|---|---|---|
| | Initial, additional Immunizing, times | Final immunizing | Immunizing number of mouse | Selected Well No. | | Measured Well Number |
| | | | | A | B | |
| 1K18 | SB cells, 3 times | Raji cells | 2 | 7 | 2 | 576 |
| 1K20 | Raji cells 3 times | SB cells | 2 | 7 | 0 | 576 |
| 1K14 | SB cells 2 times | CD20/CHO cells | 1 | 20 | 9 | 576 |
| | SB cells 3 times | CD20/CHO cells | 1 | | | |

(continued)

| Cell Fusion Series | Immunizing Method | | | Primary, Secondary Screening Specificity against CD20 on CD20/CHO cells | | |
|---|---|---|---|---|---|---|
| | Initial, additional Immunizing, times | Final immunizing | Immunizing number of mouse | Selected Well No. | | Measured Well Number |
| | | | | A | B | |
| 1K17 | CD20/CHO cells 2 times | Raji cells | 1 | 21 | >10 | 576 |
| | CD20/CHO cells 3 times | Raji cells | 1 | | | |

Selected well number-A: well reacting with CD20/CHO cells and producing antibodies not reacting with CHO cells
Selected well number-B: of the wells selected in A, well producing antibodies undergoing a competitive reaction with the reference antibody (C2B8)
The L chain variable region amino acid sequence (SEQ ID Nos: 1 to 8) and the H chain variable region amino acid sequence (SEQ ID Nos: 9 to 16) of a representative monoclonal antibody producing 8 clones is shown below.

Amino acid sequence of H chain variable region of 1K0924 (SEQ ID No: 11):

QVQLQQPGAELVKPGASVKMSCKASGYTFTSYNIHWVKQTPGQGLEWIGAIYPGNGDTS

YNQKFKGKATLTSDKSSSTAYMQLSSLTSEDSAVYYCARMSTMITGFDYWGQGTTLTVS

S

Amino acid sequence of H chain variable region of 1K1228 (SEQ ID No: 16):

QVQLQQPGAELVKPGASVKVSCKASGFTFTSYNLHWVKQTPGQGLVWIGAIYPGNGDTS

YNQKFRGKATLTADISSSTAYMQLSSLTSEDSAVYYCARYYYGYDAMDYWGQGTSVTVS

S

Amino acid sequence of H chain variable region of 1K1422 (SEQ ID No: 9):

QVQLQQPGAELVKPGASVKMSCRASGYTFTNYNMHWIKQTPGQGLEWIGAIYPGSGDTS

YNRKFKGKATLTADTSSSTAYMQFSSLTSADSAVYYCARFTYYYGGTYGAMDYWGQGTS

VTVSL

Amino acid sequence of H chain variable region of 1K1791 (SEQ ID No: 10):

QIQLVQSGPELKKPGETVKISCKASGYTFTNFGVNWVKQAPGKGLKWMGWINTYTGEPS

YADDFKGRFAFSLEASANTAYLQINNLKNDDMSTYFCTRRTNYYGTSYYYAMDYWGQGT

SVTVSS

Amino acid sequence of H chain variable region of 1K1712 (SEQ ID No: 12):

QVQLQQPGAELVKPGASVKMSCKASGFTFTSYNLHWVKQTPGQGLEWIGAIYPGSGDTS

YNQQFKGKATLTADKSSNTAYMQLNSLTSEDSAVYCCARSAMISTGNWYFDYWGQGTTL

TVSS

Amino acid sequence of H chain variable region of 1K1402 (SEQ ID No: 13):

QVQLQQPGAELVKPGASVKMSCKASGFTFTSYNMHWVKQTPGQGLEWIGGIYPGNGDTS

YNQKFKGKATLTADKSSSTAYMQLSSLTSEDSAVYYCARFYYGSMGAMDYWGQGTSVT

VSS

Amino acid sequence of H chain variable region of 1K1736 (SEQ ID No: 14):

QVQLQQPGAELVKPGASVKMSCKASGYTFTTYNLHWVKQTPGQGLEWIGGIYPGNGDTS

YNQKFKVKATLTADKSSNTAYMQLSSLTSEDSAVYYCARWIYYGNYEGTLDYWGQGTSV

TVSS

Amino acid sequence of H chain variable region of 1K1782 (SEQ ID No: 15):

QVQLQQSGAELAKPGASVKMSCKASGYTFTSYWMHWVKQRPGQGLEWIGYITPSTGYTD

YNKKFKDKATLTADRSSSTAYMHLSSLTSEDSAVYYCARSGPYFDVWGAGTTVTVSS

Amino acid sequence of H chain variable region of 1K0924 (SEQ ID No: 3):

QIVLSQSPAILSASPGEKVTMTCRASSSVSYMHWYQQRPGSSPKPWIYATSNLASGVPA

RFSGSGSGTSYYFTISRVEAEDAATYYCQQWNSNPPTHGGGTKLEIK

Amino acid sequence of H chain variable region of 1K1228 (SEQ ID No: 8):

EIILTQSPTTMAASPGEKITITCSASSSISSYYLRWYQQKPGFSPKVLIYRTSNLASGV

PARFSGSGSGTSYSLTIGTMEAEDVATYYCQQGNTVPLTFGSGTKLEIK

Amino acid sequence of L chain variable region of 1K1422 (SEQ ID No: 1):

QIVLTQSPPIMSASLGEEITLTCSASSRVSYMLWYQQKSGTSPKLLIYSTSNLASGVPS

RFSGSGSGTFYSLTISSVEAEDAADYYCHQWTSNPCTFGGGTKLEIK

Amino acid sequence of L chain variable region of 1K1791 (SEQ ID No: 2):

STVMTQTPKFLLVSAGDRVTITCKASQSVSNDVAWYQQKPGQSPKVLIYFASNRYTGVP

DRFTGSGYGTDFTFTINTVQAEDLAVYFCQQDYSSPLTFGAGTKLELK

Amino acid sequence of L chain variable region of 1K1712 (SEQ ID No: 4):

QIVLSQSPAILSASPGEKVTMTCRASSSVSYMDWYQQKPGSSPKPWIYATSNLASGVPA

RFSGSGSGTSYSLTISRVEAEDTATYYCQQWTFNPPTFGSGTKLEIK

Amino acid sequence of L chain variable region of 1K1402 (SEQ ID No: 5):

QIVLSQSPAILSASPGEKVTMTCRASSSVSYMHWYQQKPGSSPKPWIYATSNLASGVPA

RFSGSGSGTSYSLTITRVEAEDAATYYCQQWTFNPPTFGAGTKLELK

Amino acid sequence of L chain variable region of 1K1736 (SEQ ID No: 6):

QIVLSQSPAILSSSPGEKVTMTCRASSSVSYMLWYQQKPGSSPEPWIYATSNLASGVPA

RFSGGGSGTSYSLTISRVEAEDAATYYCQQWTFNPPTFGGGTKLEIK

Amino acid sequence of L chain variable region of 1K1782 (SEQ ID No: 7):

DILLTQSPAILFVSPGERVSLSCRASQNIGTSIHWYQQRTNGSPRLLIKYASESFSGIP

SRFSGSGSGTDFTLSINSVESEDIADYYCQQSNSWPFTFGSGTKLEIK

EXAMPLE 2

The base sequence of the variable region of the monoclonal antibody gene was determined for a portion of the resulting clones. Measurements of the antibody binding affinity and biological characteristic tests for monoclonals producing the sequences are described hereinafter.

(1) Binding Affinity Measurement

Floating Raji cells derived from human B cells expressing the target antigen on the cellular surface and floating Jurkat cells derived from human T cells not expressing CD20 antigen were used. The cells were cultured in a $CO_2$ incubator (SANYO MCO-175M) at 37°C in an atmosphere of 5% $CO_2$ using RPMI1640 medium (nacalai tesque Co., Ltd., Cat.No.30264-85, Lot L4K2844) supplemented with 10% fetal calf serum (FCS) (BIOLOGICAL IND. Cat.No. 04-001-1A, Lot 815242, a preparation which is preheated at 56°C for 30 minutes in order to deactivate the complement component). The cells were maintained by subculturing twice per week.

Measurement of cell numbers was performed using a Burker-Turk hemacytometer (Erma, Inc., Cat. No. 03-303-1). Confluent-cell culture medium three to four days after subculturing was centrifuged for three minutes at 3000 rpm at room temperature using a multipurpose refrigerated centrifuge LX-120 (TOMY Co. Ltd.). The supernatant was removed and the cells were recovered. The rotation speed and time used in this step were selected so that the number of cells displayed no change irrespective of the repetition of centrifugal separation and removal of the supernatant. In order to remove culturing medium and FCS remaining on the cell surface (washing), the recovered cells were suspended in Dulbecco's Phosphate Buffered Saline (-) (free of Ca and Mg, PBS(-), (NaCl : Wako, Cat. No. 191-01665, $Na_2HPO_4$ : Wako, Cat.No.197-02865, Lot ASF2635, KCl : Wako, Cat.No. 163-0334T, Lot CEQ7122, $KH_2PO_4$ : Wako, Cat. No. 169-0425, Lot ELG7616)) and centrifuged twice for 3 minutes at 3000 rpm in order to remove supernatant. Cells after washing were suspended in a solution of 1% BSA (Wako Cat No. 013-07492 Lot PKH3483) - PBS and adjusted to a cell density of $5 \times 10^6$ cells/ml.

A primary antibody, which is a test antibody or a positive control antibody (2B8), was respectively injected in 15, 30, 50, 75, 100, 125, 150, 200 ng (1.5 to 5 $\mu$l) lots into a 1.5 ml tube (BM Equipment Co., Ltd., BM-ring lock tube, Cat. No. BM-15). At the same time, four tubes not containing antibody were prepared. Three samples were prepared for each test antibody, mixed well with 100 $\mu$l ($5 \times 10^5$ cells) of a suspension of 1% BSA (Wako Cat No. 013-07492 Lot PKH3483) - PBS, and shaken and reacted at room temperature for one hour.

After reacting, centrifugal separation was performed at 3,000 rpm for 3 minutes at room temperature using a low-temperature high-speed refrigerated centrifuge LX-100 (TOMY). After recovering the cells, the cells were suspended in 200 $\mu$l of PBS to remove unreacted primary antibody remaining on the cell surface and then centrifuged at 3,000 rpm for three minutes to remove the supernatant. This operation was repeated twice.

FITC-labeled anti murine IgG (H&L) secondary antibody [GOAT Anti-murine IgG (H&L) Fluorescein conjugated, affinity purified Secondary antibody, Chemicon, Cat. No. AP124F, Lot 24021014] was added in excess (500 ng) with respect to cell-conjugated primary antibody together with 100 $\mu$l of 1% BSA-PBS (500 ng/100 $\mu$l). The mixture was shaken for one hour at room temperature while shielding against light and primary antibodies binding to cells were detected. After the reaction, the mixture was centrifuged at 3,000 rpm for three minutes and the cells were recovered. The cells were suspended in 200 $\mu$l of PBS to remove unreacted FITC-labeled anti murine IgG (H&L) antibody remaining on the cell surface and then centrifuged at 3,000 rpm for three minutes to remove the supernatant. This operation was repeated twice.

The recovered cells were suspended in 100 $\mu$l of PBS and transferred to a flat-bottomed 96 well plate (Sumitomo Bakelite Co., Ltd., ELISA PLATE Cat. No. 8496F). The fluorescent intensity of the secondary antibodies was measured using a Typhoon9210 image analyzer (Amersham Bioscience) under the following conditions: Fluorescence mode: 600 V, 526SP/green(532 nm), Focus : bottom face + 3 mm. At the same time, the controls for the preparation of a standard curve were prepared using 100 $\mu$l of PBS supplemented with 0, 12.5, 25, 50, 75, 100, 125, 150 ng of FITC-labeled secondary antibody.

After the detection step, the image was digitized using image analysis software Image Quant (Amersham Bioscience) and analyzed using Excel (Microsoft).

Background values for the plate, the PBS solution and FITC-labeled secondary antibody displaying non-specific binding to cells were determined. Then a value for the reaction only between the cells and the FITC-labeled secondary antibodies was obtained. Average values for those four points were subtracted from the fluorescent intensity value for each sample in order to obtain the amount of fluorescence of cell-conjugated FITC-labeled secondary antibody. A standard curve was prepared by measuring the amount of fluorescence at various concentrations of control cell-conjugated FITC-labeled secondary antibodies. Thus the amount of cell-conjugated secondary antibody (number of moles or weight) was calculated. The amount of cell-conjugated primary antibody was calculated assuming that each primary antibody and the FITC-labeled secondary antibody react at a ratio of 1:2. Primary antibody in suspension was calculated by subtracting the cell-conjugated amount from the added amount. When calculating the antibody concentration as a molar concentration, the molecular weight of the monoclonal was taken to be 150,000.

Saturation of the binding reaction resulting from increasing addition of primary antibody was confirmed when the fluorescent intensity reached a constant value. Scatchard analysis was used to calculate the antigen number and dissociation constant (Kd value) refer to Scatchard, G.; Ann. N.Y. Acad. Sci.,51: 660-672,1949, New Cultured Cell Experimental Methods in Molecular Biology Research, Yodosha Co., Ltd., Jikken Igaku separate volume, BioManual UP Series Revised 2nd Edition, pages 212 to 217). The values were an average of three values for each sample. The measurement results for an example of a representative monoclonal antibody producing 8 clones and a positive control antibody (2B8) are shown below in Table 3.

(2) Biological Characteristics Tests

(a) Apoptosis Induction Test

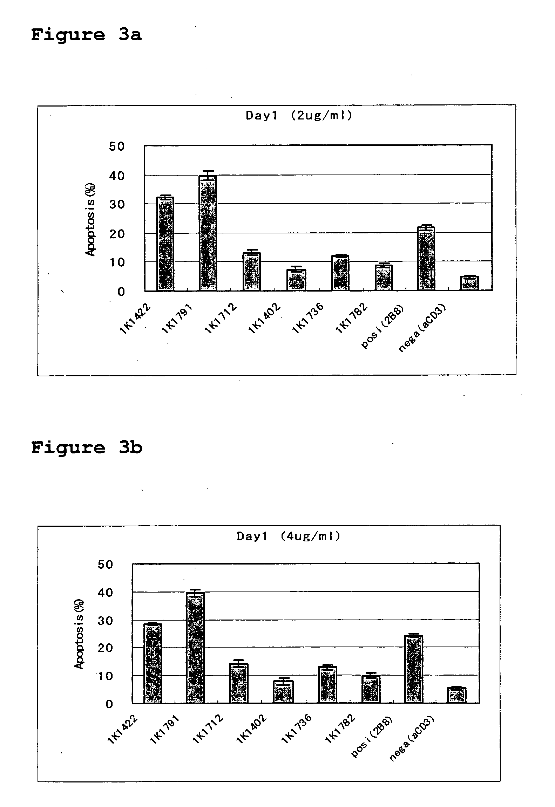

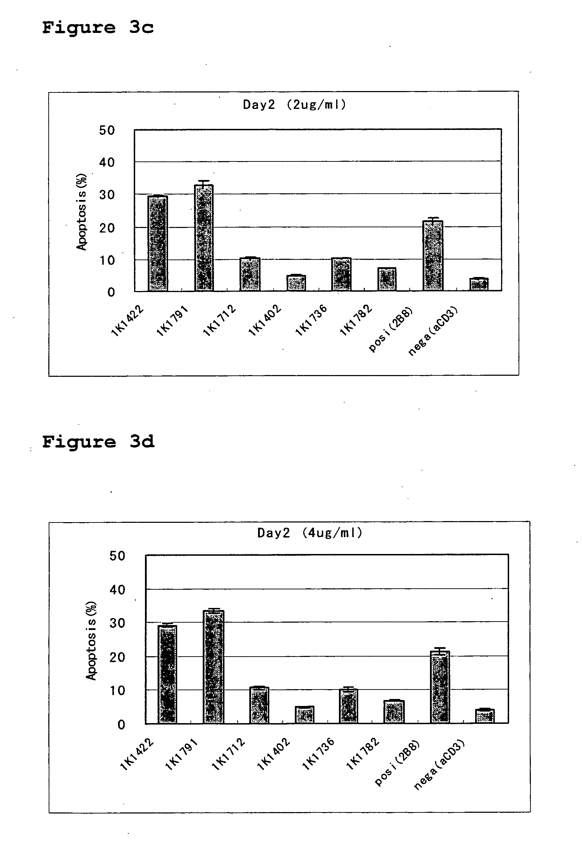

The apoptosis induction capacity of a test antibody was measured using flow cytometry (Annexin V/ PI staining). A positive control (2B8) and a negative control (Anti-CD3 monoclonal antibody (BD PharMingen) were used. The test was performed using a MEBCYTO Apoptosis Kit (MBL, Cat. No. 4700, Lot. 20). After centrifuging, Raji cells were suspended in fresh RPMI1640 medium (Sigma, Cat.No.R8758, Lot.44K2416) supplemented with 10% FBS (immobilization agent) (ICN, Cat. No. 2916754, Lot. 8005C). 1 ml of the solution with a concentration $5 \times 10^5$ cells/ml was introduced to each well of a 12-wellplate. 12 wells were used for each antibody and each antibody was added to make a final concentration of 2 $\mu$g/ml or 4 $\mu$g/ml (3 wells x two concentrations x 2 times, total 12 wells). On the first and second day after commencement of culturing, culture medium containing about $2 \times 10^5$ cells was recovered and after centrifuging, the cells were washed once in PBS. Then the cells were suspended in 85 $\mu$l of binding buffer. After mixing well with 10 $\mu$l of Annexin V-FITC and 5 $\mu$l of PI, the mixture was allowed to react for 15 minutes at room temperature while shielding from the light. Flow cytometry measurements were performed (FACS Calibur, Becton Dickinson) and analyzed CellQuest (Becton Dickinson). The measurement results for an example of a representative monoclonal antibody producing 6 clones, a positive control antibody (2B8) and a negative control antibody (Anti-CD3) are shown below in Fig. 3a to Fig. 3d. Although generally 28B was expected to have high apoptosis inducing capacity, the monoclonal antibody producing the clone 1k1791 obtained by cell fusion of the 1K17 series (immunized with.CD20/CHO and Raji cells) and the clone 1k1422 obtained by cell fusion of the 1K14 series (immunized with SB cells and CD20/CHO cells) displayed a high apoptosis inducing capacity when compared with 2B8.

(b) Cell Growth Inhibiting Tests

A Raji cell suspension having a cell concentration of $5 \times 10^4$ cells/ml was supplemented with RPMI1640 supplemented with 10% FCS. The resulting solution was added in 100 $\mu$l/well lots to a 96-wellplate and cultured. After 24 hours, culturing was continued after adding 50 $\mu$l/well of the respective antibody solutions to each well so that the antibody concentration was 1 $\mu$l/ml. 72 hours after adding the antibody, 10 $\mu$l/well of color development Cell Counting Kit-8 (Donin Kagaku, Cat. No. 343-07623, Lot. SG076) was added, culturing was continued for another 4 hours and then absorption was measured at 492 nm. The absorbance measurement results for a monoclonal antibody producing 6 clones, a positive control antibody (2B8) and a negative control are shown below in Table 2 and their characteristics are shown in Table 3.

Cell Growth Inhibiting Tests

Table 2

| Clone name | Absorption (492 nm) |
|---|---|
| 1K1422 | 1.775 |
| IK1791 | 1.794 |
| IK1712 | 2.326 |
| 1K1402 | 2.540 |
| 1K1736 | 2.239 |
| 1K1782 | 2.603 |
| Positive control (2B8) | 1.759 |
| Negative control | 2.607 |

Characteristics of Monoclonal Antibodies

Table 3

| Clone name | Isotype | Binding affinity Kd value (nM) | Capacity to Induce Apoptosis | Cell Growth Inhibiting Action (in Vitro) |
|---|---|---|---|---|
| 1K1422 | IgG1, κ | 3.39 | 130 | present |
| 1K1791 | IgG1, κ | 1.70 | 160 | present |
| 1K0924 | IgG2b, κ | 1.35 | 60 | present |
| 1K1712 | IgG2a, κ | 0.84 | 50 | - |
| 1K1402 | IgG1, κ | 0.78 | 30 | - |
| 1K1736 | IgG2b, κ | 0.54 | 50 | - |
| 1K1782 | IgG1, κ | 0.40 | 30 | - |
| 1K1228 | IgG1, κ | 0.26 | 30 | - |
| Positive control (2B8) | IgG1, κ | 6.79 | 100 | present |

(c) Antibody-Dependent Cell-Mediated Cytotoxicity (ADCC)

In this experiment, effector cells were activated using the Fc region of an anti CD20 chimerized antibody in order to measure the capability to lyse lymphoma cell lines.
Four types of cells, Raji, WiL2-NS, SU-DHL4 and RC-K8 derived from human B cells were cultured and incubated in RPMI1640 supplemented with inactivated 10% FCS (culturing medium) at 37°C under an atmosphere of 5% $CO_2$. During the experiment, the cells were washed in RPMI1640 supplemented with 10% FCS. Untreated cells took up calcein for 15 minutes during the reaction at 37°C and then the number of cells was adjusted to 4 x $10^5$ cells/ml. Since calcein is only found on cells having a normal cellular membrane, it is possible to stain only normal cells. Rituximab (C2B8) which is an anti CD20 chimerized antibody and the 6 types of chimerized antibody (1k0924, 1k1402, 1k1422, 1k1712, 1k1736, 1k1791) were adjusted to 20 μg/ml, 4 μg/ml and 0.8 μg/ml using RPMI1640 supplemented with 10% FCS. Blood was taken from a healthy subject immediately layered onto a Ficoll and centrifuged in order to extract a lymphocyte fraction which was then adjusted to 5 x $10^6$ cells/ml, 1 x $10^6$ cells/ml and 0.2 x $10^6$ cells/ml.
25 μl of cells with an adjusted concentration, 25 μl of each anti CD20 chimerized antibody solution (having respective final concentrations of 5 μg/ml, 1 μg/ml, 0.2 μg/ml) and 50 μl of effector cells at each antibody concentration (having respective E:T ratios of 25:1, 5:1 and 1:1) making a total of 100 μl was mixed well in a 96-wellplate and reacted for 4 hours in an incubator at 37°C under an atmosphere of 5% $CO_2$. In order to calculate the natural lysis of cells, three samples were prepared: a sample in which the antibody solution and the effector cells are replaced by RPMI1640 supplemented by 10% FCS, a sample in which the antibody solution is replaced by RPMI1640 supplemented by 10% FCS which is a sample for calculating the activity of only the effector cells independently of the presence of antibodies, and a sample in which the antibody solution is replaced by 20% TritonX-100 which is a sample for calculating the maximum lysis.
After the reaction, since calcein is discharged out of the cells as a result of the rupture of the cellular membrane when the cell lyses, Quencher was used to quench the fluorescence of calcein suspended in the reaction solution and then fluorescence was measured using a fluorescence analyzer.
After analysis, the image was digitized using image analysis software (Amersham Bioscience) in order to calculate a lysis rate for each sample using the equation below.

```
Equation 1

Lysis rate % = ((Natural lysis) - (Sample))/((natural

lysis) - (Maximum lysis)) x 100
```

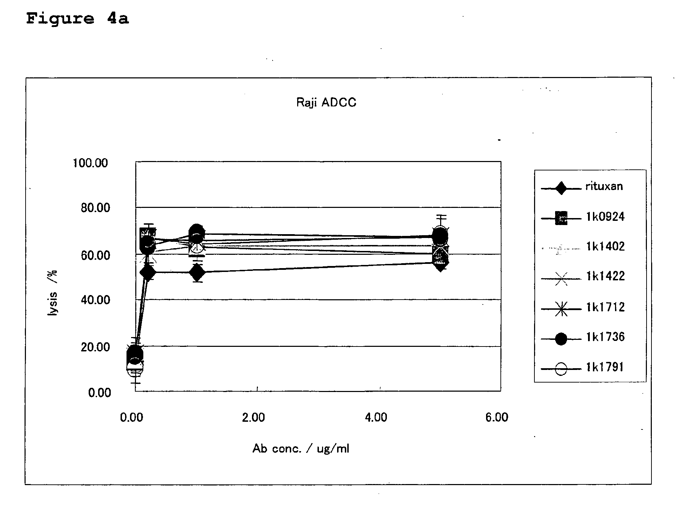

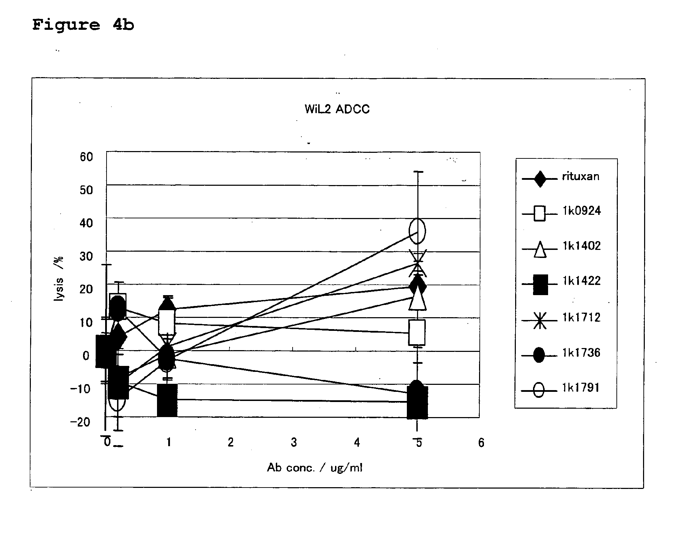

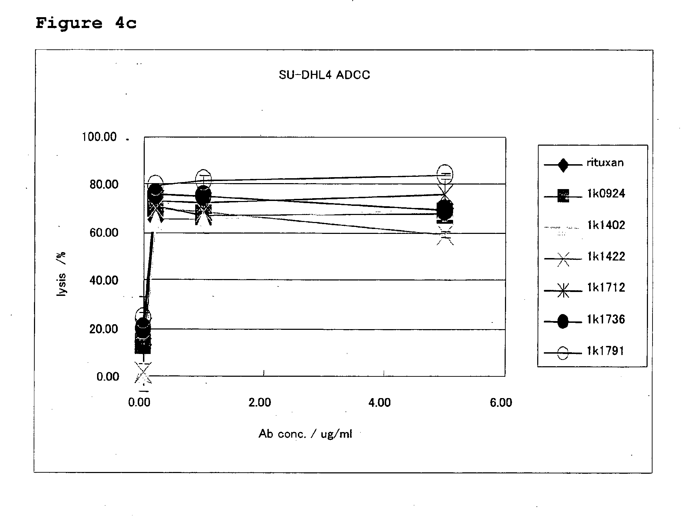

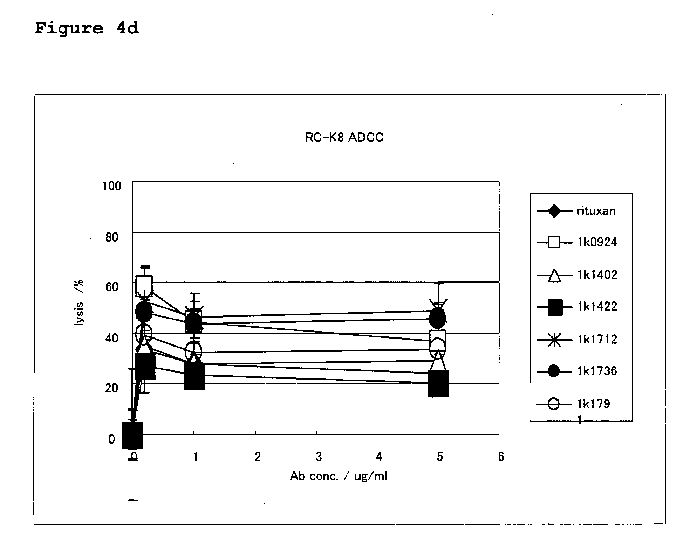

The relationship between antibody concentration and cytotoxicity for each cell type when the ratio (E:T ratio) of the number of target cells to the number of effector cells such as NK cells is 25 : 1 is shown in Fig. 4a to Fig. 4d. The relationship between cytotoxicity and the E:T ratio for each cell type when the antibody concentration is 5 μg/ml is shown

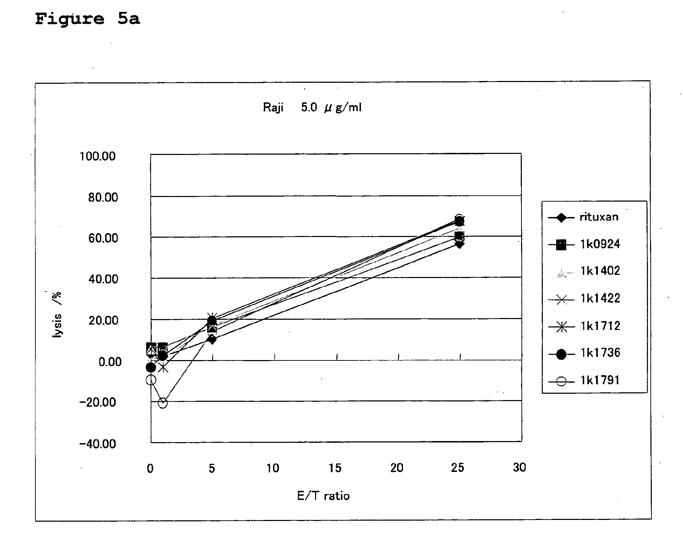

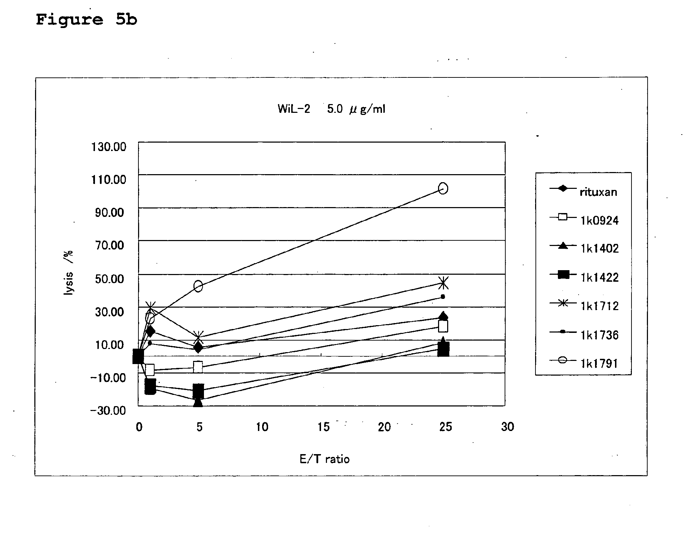

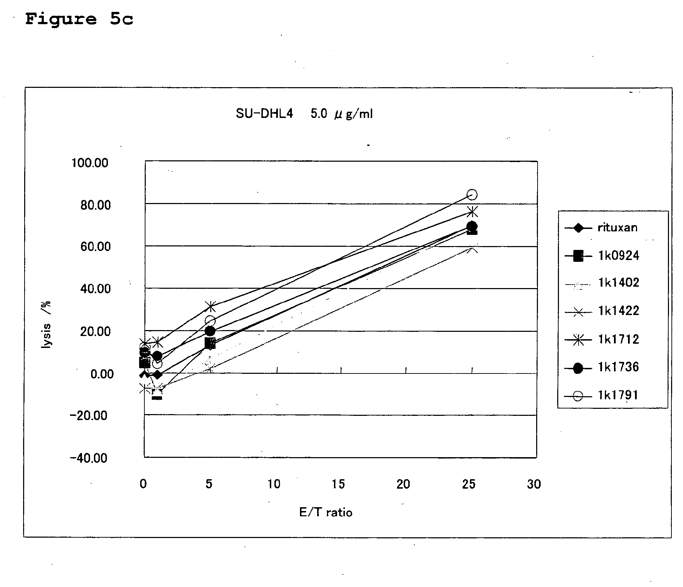

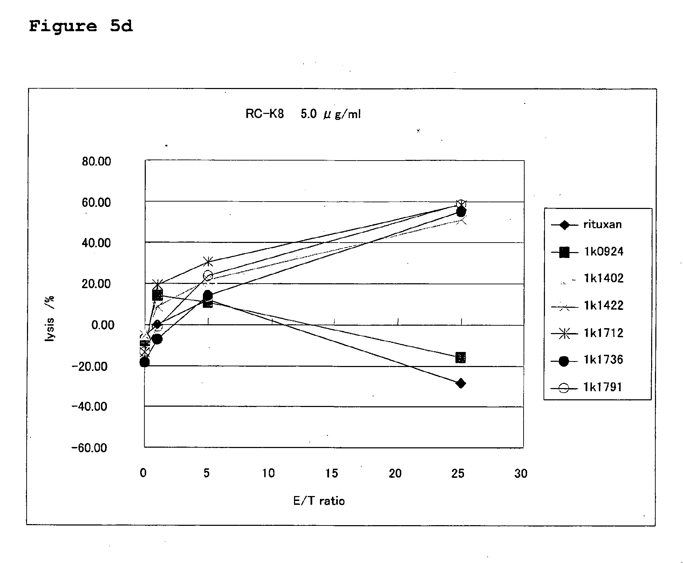

in Fig. 5a to Fig. 5d.

As shown in Fig. 4a to Fig. 4d, when the E:T ratio is 25 : 1, each cell shows cytotoxicity when antibodies are added. In other words, the antibodies participate in cytotoxicity. Furthermore cell strains other than WiL2-NS display activity equivalent to 1 $\mu$g/ml and 5 $\mu$g/ml at an antibody concentration of 0.2 $\mu$g/ml (saturation occurs at 0.2 $\mu$g/ml). Activity becomes constant after reaching a maximum and effects are evident at an antibody amount which is less than the antibody required for complement dependent cytotoxicity.

As shown in Fig. 5a to Fig. 5d, the effect of the E:T ratio on cytotoxicity when the antibody concentration is 5 $\mu$g/ml is that cytotoxicity increases in an E:T ratio-dependent manner. This shows that cytotoxicity occurs as a result of effector cell action.

(d) Complement Dependent Cytotoxicity (CDC)

In this experiment, measurements were made of the capability of anti CD20 chimerized antibodies to lyse lymphoma cell lines in the presence of serum containing complement.

Four types of cells, Raji, WiL2-NS, SU-DHL4 and RC-K8 derived from human B cells were cultured and incubated in RPMI1640 supplemented with inactivated 10% FCS (culturing medium) at 37°C under an atmosphere of 5% $CO_2$. During the experiment, the cells were washed in RPMI1640 supplemented with 10% FCS and the cell number was adjusted to 2-3 x $10^6$ cells/ml. C2B8 (rituximab) which is an anti CD20 chimerized antibody and the 6 types of chimerized antibody (1k0924, 1k1402, 1k1422, 1k1712, 1k1736, 1k1791) were adjusted to 20 $\mu$g/ml, 4 $\mu$g/ml and 0.8 $\mu$g/ml using RPMI1640 supplemented with 10% FCS.

55 $\mu$l of cells with an adjusted concentration, 25 $\mu$l of each anti CD20 chimerized antibody solution (having respective final concentrations of 5 $\mu$g/ml, 1 $\mu$g/ml, 0.2 $\mu$g/ml) and 20 $\mu$l of pooled serum taken from five healthy subject or an inactivated type thereof making a total of 100 $\mu$l was mixed well using a vortex mixer and reacted for 2 hours in an incubator at 37°C under an atmosphere of 5% $CO_2$. A sample in which 25 $\mu$l of antibody solution is replaced by RPMI1640 supplemented with 10% FCS was prepared as a sample for background calculations.

After the reaction, PI (propidium iodide) was used to stain dead cells and analysis was performed using a FACS (Becton Dickinson). The numerical results used the population of dead cells without modification and subtracted the background values and the sample supplemented with inactivated serum.

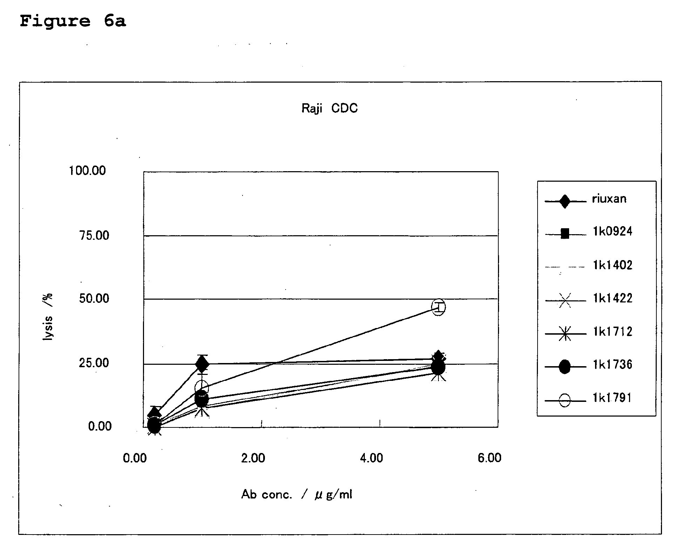

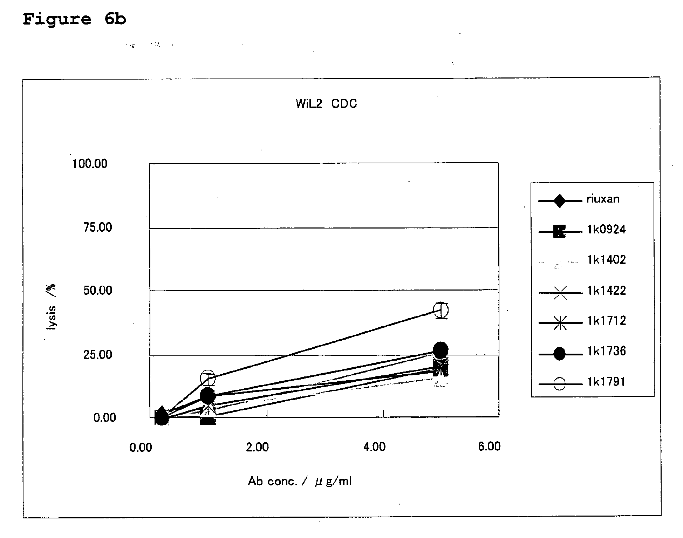

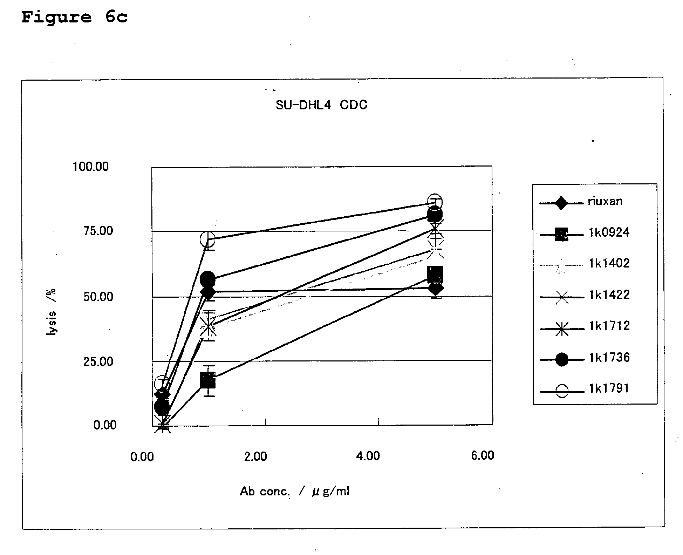

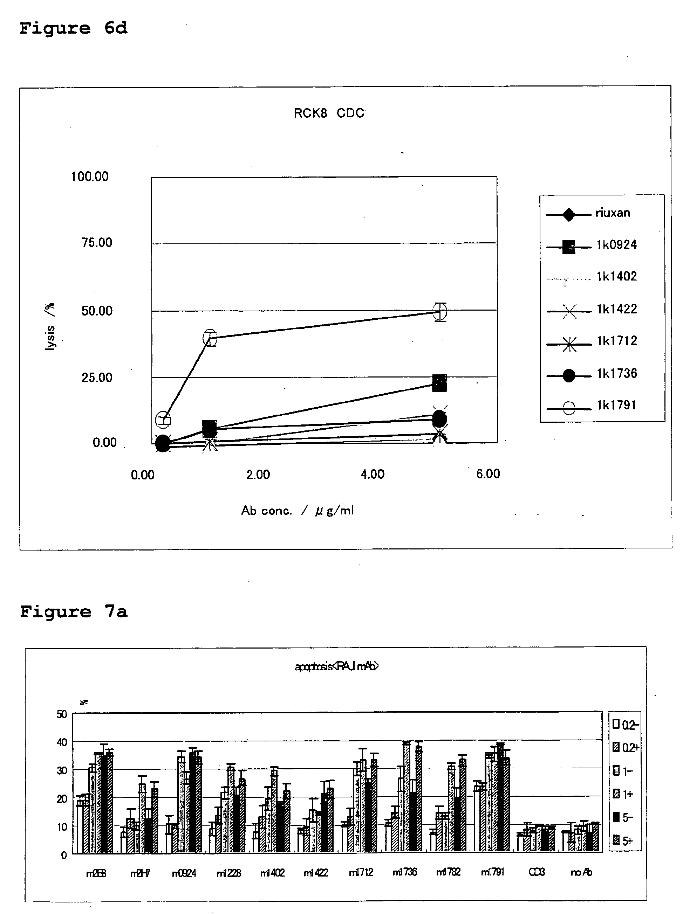

The relationship between antibody concentration and cytotoxicity for each cell type is shown in Fig. 6a to Fig. 6d.

As shown in Fig. 6a to Fig. 6d, all six types of antibodies show activity in Raji, WiL2-NS and SU-DHL4. Furthermore, although concentration dependency can be confirmed, when concentrations at 5 $\mu$g/ml are compared, 1k1791 shows a particularly high activity in comparison to the other antibodies. In addition, 1k1736, 1k1422 and 1k1712 show high activity. At this concentration, 1k1791 induces approximately twice the cytotoxicity with respect to WiL2-NS cells in comparison to other antibodies. However the other antibodies also display equal or greater activity than rituximab.

RC-K8 cells on which rituximab has no effect shows very clearly the difference between the respective antibodies. Rituximab, 1k1402 and 1k1712 show no activity or almost no activity. In contrast, 1k1791 shows extremely high activity, and at a concentration of 5 $\mu$g/ml shows cytotoxicity of approximately 50%. Thereinafter 1k0924 shows cytotoxicity of approximately 25% and 1k1422 and 1k1736 show cytotoxicity of approximately 10%.

From the above, it can be confirmed that the 6 types of chimerized antibodies which are the subject of the present test display CDC activity which is equal to or stronger than rituximab.

EXAMPLE 3

(1) Measurement of Binding Affinity

Raji cells derived from human B cells were cultured in a $CO_2$ incubator at 37°C in an atmosphere of 5% $CO_2$ using RPMI1640 medium supplemented with inactivated 10% FCS. The cells were passaged twice per week.

Culture solution on three to four days after subculturing containing (approximately 1 x $10^6$ cells/ml) cells was centrifuged for five minutes at 1,000 rpm at room temperature. The cells were recovered, suspended in PBS (-) and centrifuged for five minutes at 1,000 rpm in order to remove supernatant. This operation was performed twice and then the cells were washed.

The primary antibody reaction was performed by mixing well an anti CD20 antibody (positive control antibody: antibody 2B8, chimerized antibody and humanized antibody C2B8) with Raji cells and reacting the mixture at room temperature for one hour. The respective final concentrations of anti CD20 antibodies has twelve values of 1.33, 2.67, 4.00, 5.33, 6.67, 8.00, 9.33, 10.67, 12.00, 13.33, 14.67, 16.00 nM. The reaction solution was 1% BSA-PBS solution with a cell number of 5 x $10^6$ cells and was injected into a 1.5 ml tube to a final volume of 100 $\mu$l. Three samples for each test antibody were prepared and four tubes to which antibody was not added were prepared as samples for background calculations.

After reacting, the mixture was centrifuged at room temperature for three minutes at 3,000 rpm in order to remove unreacted primary antibody and the cells were recovered.

FITC-labeled secondary antibody was adjusted to have a concentration of 5 $\mu$g/ml in 1% BSA-PBS solution. This solution was added in 100 $\mu$l lots so as to be in excess with respect to the primary antibody binding to the cells. After suspending and shielding against the light, the solution was reacted for one hour at room temperature.

When the FITC-labeled secondary antibody used was a murine antibody, the antibody was GOAT Anti-murine IgG (H&L)-FITC. When the secondary antibody was a chimerized or humanized antibody, the antibody was GOAT F(ab') 2 Fragment Anti Human IgG (Fc$\gamma$)-FITC.

After the reaction, the mixture was centrifuged at 3,000 rpm for three minutes at room temperature. Unreacted FITC-labeled secondary antibody was removed and the cells were recovered. The cells were suspended in 200 $\mu$l of PBS centrifuged again and washed.

The cells were suspended in 100 $\mu$l of PBS and transferred to a flat-bottomed 96-well plate. The fluorescent intensity of the secondary antibodies was measured using a Typhoon9210 analyzer (Amersham Bioscience).

After the detecting step, an image was digitized using image analysis software Image Quant (Amersham Bioscience) and analyzed using Excel (Microsoft). Average values for the same test antibodies were calculated and the values for background calculation samples (when only reacting a cell with a FITC-labeled secondary antibody) were subtracted from the test antibody values. The background values derived for the FITC-labeled secondary antibody binding non-specifically to cells, the PBS solution and the plate are omitted. At the same time, a standard curve was prepared by measuring the amount of fluorescence of only FITC-labeled secondary antibodies at amounts of 0, 12.5, 25, 50, 75, 100, 125, and 150 ng per 100 $\mu$l. In this manner, the number of moles of secondary antibody binding to cells was calculated. Assuming that each primary antibody and the FITC-labeled secondary antibody react at a ratio of 1:5, the amount of cell-bound primary antibody was calculated. Primary antibody in suspension was calculated by subtracting the bound amount from the added amount. Scatchard analysis was performed using these values in order to calculate a dissociation constant Kd.

The results are shown below in Table 4.

(2) Apoptosis Inducing Test

Initial apoptosis was detected using a Annexin V-FITC apoptosis kit using two conditions: a condition (no cross linking) in which apoptosis induced independently by anti CD20 antibodies against cells derived from human B cells stains, and a condition (cross linking) in which apoptosis was induced by adding secondary antibodies recognizing the Fc region of the anti CD20 antibody.

Four types of cells, Raji, WiL2-NS, SU-DHL4 and RC-K8 derived from human B cells were cultured by incubating in RPMI1640 supplemented with inactivated 10% FCS (culturing medium) at 37°C under an atmosphere of 5% $CO_2$. The cells were subcultured twice per week.

Three to four days after subculturing, culture solution (approximately 1 x $10^6$ cells/ml) was centrifuged for five minutes at 1,000 rpm at room temperature and the cells were recovered.

Anti CD20 antibodies (positive control antibody: murine antibody 2B8, chimerized antibody and humanized antibody C2B8, negative control: Anti-CD2 monoclonal antibody) were mixed well with cells suspended in fresh culture medium and reacted in an incubator at 37°C under an atmosphere of 5% $CO_2$. The final concentrations of the anti CD20 antibodies were 0.2, 1 and 5 $\mu$g/ml. The culture medium with a cell concentration of 1 x $10^6$ cells was used as the reaction solution and was reacted in a 1.5 ml tube to a final volume of 250 $\mu$l. Three samples were prepared for each test antibody.

After the reaction, the mixture was centrifuged at 1,200 rpm for three minutes at room temperature, unreacted antibody was removed and the cells were recovered.

Under the no cross linking condition, fresh culturing media was used, under the cross linking condition, five times the amount of the CD20 antibody of a secondary antibody recognizing the Fc region was added in 250 $\mu$l lots. After mixing well, the mixture was reacted for three more hours in an incubator at 37°C in an atmosphere of 5% $CO_2$. When secondary antibody used was a murine antibody, the antibody was GOAT Anti-murine IgG Fc$\gamma$ Fragment and when the secondary antibody was a chimerized or humanized antibody, the antibody was GOAT Anti Human IgG Fc$\gamma$-Fragment specific.

After the reaction, the mixture was centrifuged at 3,000 rpm for three minutes at room temperature. Unreacted secondary antibody was removed and the cells were recovered. The cells were suspended in 250 $\mu$l of PBS, centrifuged again and washed.

Test reagents from a MEBCYTO apoptosis kit-AnnexinV-FITC, PI- (MBL, Cat. No. 4700, Lot. 21) were used. After suspending the cells in 85 $\mu$l of Binding buffer, 5 $\mu$l of Annexin V-FITC and 5 $\mu$l of propidium iodide (PI) (to a final concentration of 0.5 mg/ml) were added and mixed well. The mixture was shielded from the light and allowed to react at 15 minutes at room temperature.

A total count 20,000 of cells was measured using flow cytometry (EPICS ALTRA :BECKMAN COULTER) and analyzed (Expo32: BECKMAN COULTER).

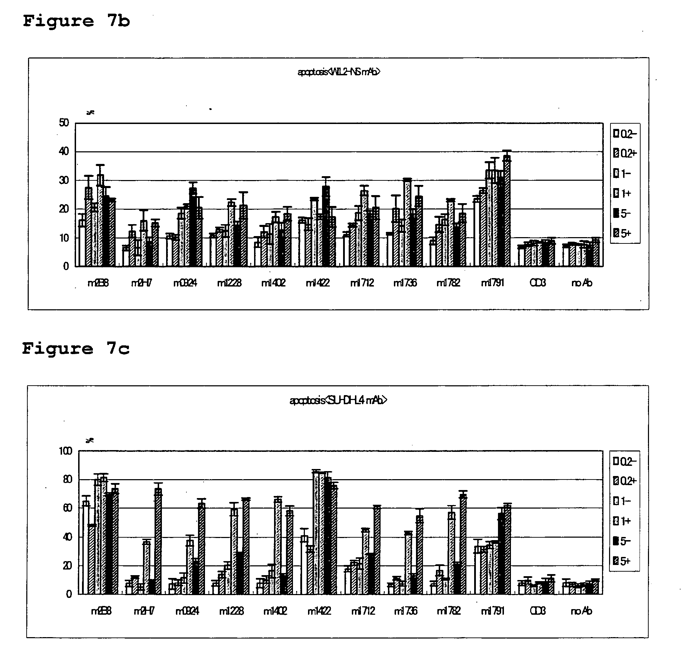

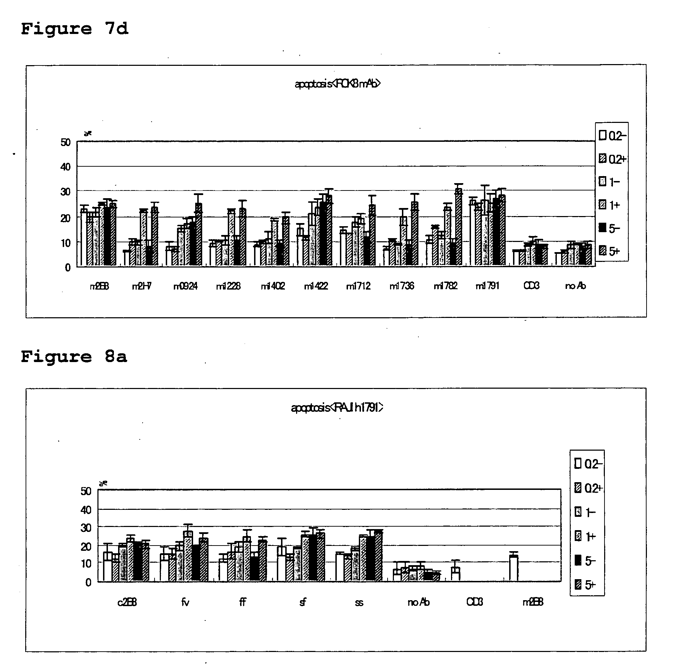

The results are shown in Fig. 7a to Fig. 9d.

(3) Preparation of Humanized Antibody Producing Strain

(a) DNA Synthesis

DNA optimized to CHO cells with a codon based on amino acid sequences in SEQ ID Nos: 17 to 24 were designed and synthesized.

(b) Preparation of the Construct

16 types of humanized 1K1791 expression constructs were prepared using pNOW as an expression vector. pNOW-aa1791kg1, pNOW-af1791kg1, pNOW-as1791kg1, pNOW-av1791kg1, pNOW-fa1791kg1, pNOW-ff1791kg1, pNOW-fs1791kg1, pNOW-fv1791kg1, pNOW-sa1791kg1, pNOW-sf1791kg1, pNOW-ss1791kg1, pNOW-sv1791kg1, pNOW-va1791kg1, pNOW-vf1791kg1, pNOW-vs1791kg1, pNOW-vv1791kg1

(c) Transfection and selection using chemical reagent

A humanized 1K1791 expression construct was introduced into CHO DG44cdB cells using a transfection reagent. 1 x 10$^6$ CHO DG44cdB cells containing the respective genes were suspended in 100 μl of selective medium, dispersed on five 96-wellplates (200 μl/well) and cultured for 3 to 4 weeks at 37°C under an atmosphere of carbon dioxide gas.
Transfection reagent: Qiagen, Effectene Transfection Reagent, Cat. No 301427.
Selective Medium: IS CHO-CD w/Hydrolysate/4mM GlutaMAX/0.8 mg/ml G418.

(d) Selection of High Expression Cell Strain

1) Supernatant was recovered from wells where colonies were present and the antibody production amount was measured using a Dot Blot assay.
2) Clones displaying a high antibody production amount were transferred to a 24 wellplate and after culturing for approximately 5 days, the supernatant was recovered and the antibody production amount was measured using a Sandwich ELISA
3) Two clones displaying a high antibody production amount were selected and transferred to a T75 flask.

(e) Small Scale Culturing

The two selected clones were cultured in a T75 flask containing 30 μl of selective medium against the 16 types of constructs.

(4) Culturing and Purification of Humanized Antibody Producing Strains

Antibody producing cell strains (genetically recombinant CHO-DG44 cells) were cultured in IS CHO-CD/with Hydrolysate medium (Irvine Scientific, Cat. No. 91119) containing Hydrolysate supplemented with 4 nM GlutaMax (Invitrogen, Cat 35050-061) and 200 μg/ml of G418 (Sigma, Cat. No. A1720-5G) in a $CO_2$ incubator under an atmosphere of 5% $CO_2$ at 37°C. The cells were passaged twice per week.
Cell culture solution approximately two weeks after subculturing was centrifuged at 3,500 rpm for five minutes at room temperature. The supernatant was recovered, filtered using a 0.45 μm syringe filter and equilibrated using 50 nM Tris-HCl, pH 7.0.
After adding supernatant to a Hi Trap Protein A HP column (GE Healthcare, Cat No. 17-0402-01), washing was performed using 50 nM Tris-HCl, pH 7.0. Elutions were obtained using 0.1M citric acid pH 4.0. 400 μl was collected on each occasion and neutralized with 40 μl (or a 10/1 amount) of 1 M Tris-HCl, pH 9.0. After dialyzing twice against a 100 times amount of PBS for 2.5 hours using a M. W. 3500 diafiltration cup (Bio-Tech Cat. No. 212932), dialysis was performed for 15 to 18 hours once.
The antibody producing strains used were CHO cells hz1791-fv10, hz1791-ff34, hz1791-sf43 and hz1791-ss32. These strains were deposited respectively under Accession Nos. FERM BP-10543, FERM BP-10544 FERM BP-10545 FERM BP-10546 with the International Patent Organism Depositary (IPOD), National Institute of Advanced Industrial Science and Technology (AIST), Tsukuba Central 6, 1-1-1 Higashi, Tsukuba, Ibaraki, Japan on 1 March 2006 under the provisions of the Budapest Treaty.

The amino acid sequences (SEQ ID Nos: 17 to 24) for the H chains of the variable region and the L chain of the variable region of a humanized anti CD20 monoclonal antibody obtained from the above cell strains are described hereinafter (the underlined sections differ from the corresponding murine antibody). Sequence for Humanized 1k1791

Amino acid sequence of L chain variable region (SEQ ID No: 20)

Ven 1791 :STVMTQSPDSLAVSLGERVTINC KASQSVSNDVA

WYQQKPGQSPKVLIY FASNRYT GVPDRFSGSGYGTDFTFTISSVQAEDVAVYFC

QQDYSSPLT FGAGTKLELK

Amino acid sequence of H chain variable region (SEQ ID No: 24)

Ven 1791 : QIQLVQSGPELKKPGASVKISCKASGYTFT NFGVN

WVKQAPGKGLKWMG WINTYTGEPSYADDFKG RFAFSLDASVSTAY

LQISSLKAEDTSTYFCTR RTNYYGTSYYYAMDY WGQGTTVTVSS

Amino acid sequence of L chain variable region (SEQ ID No: 17)

abb 1791 :STVMTQSPDSLAVSLGERATINC KSSQSVSNDVA

WYQQKPGQSPKVLIY FASNRYS GVPDRFSGSGYGTDFTLTISSLQAEDVAVYFC

QQDYSSPLT FGAGTKLEIK

Amino acid sequence of H chain variable region (SEQ ID No: 21)

abb 1791 : QIQLVQSGSELKKPGASVKVSCKASGYTFT NFGVN

WVRQAPGKGLEWMG WINTYTGEPSYAQGFTG RFVFSLDASVSTAY

LQISSLKAEDTATYFCTR RTNYYGTSYYYAMDY WGQGTTVTVSS

Amino acid sequence of L chain variable region (SEQ ID No: 19)

sdr 1791 : STVMTQSPDSLAVSLGERATINC KSSQSNSNDVA

WYQQKPGQSPKVLIY FASNRYS GVPDRFSGSGYGTDFTLTISSLQAEDVAVYFC

QQDYSSPLT FGAGTKLEIK

Amino acid sequence of H chain variable region (SEQ ID No: 23)

```
sdr 1791 : QIQLVQSGSELKKPGASVKVSCKASGYTFT NFGVN

WVRQAPGKGLKWMG WINTYTGEPSYAQGFTG RFAFSLDASVSTAY

LQISSLKAEDTATYFCTR RTNYYGTSYYYAMDY WGQGTTVTVSS
```

Amino acid sequence of L chain variable region (SEQ ID No: 18)

```
fra 1791 : STVMTQSPSFLSASVGDRVTITC KASQSVSNDVA

WYQQKPGQSPKVLIY FASNRYT GVPDRFSGSGYGTDFTLTISSLQAEDVAVYFC

QQDYSSPLT FGAGTKLEIK
```

Amino acid sequence of H chain variable region (SEQ ID No: 22)

```
fra 1791 : QIQLVQSGSELKKPGASVKVSCKASGYTFT NFGVN

WVKQAPGKGLKWMG WINTYTGEPSYADDFKG RFAFSLDASASTAY

LQISSLKAEDMATYFCTR RTNYYGTSYYYAMDY WGQGTTVTVSS
```

In this experiment, average values were obtained by performing experiments with respect to each two clones from the total combination of 16 types of sequences resulting from combining the four types AbbL, FraL, SdrL, VenL x the four types AbbH, FraH, SdrH, VenH. From the above results, it is possible to create a classification of 4 groups as shown in Table 4 based on Kd values. One type was selected from each group and used in the experiment described hereinafter.

Control: c2B8

Group I: Kd = 20 nM <

Group II: Kd = 10 to 20 nM

Group III: Kd = 8 to 10 nM

Group IV: Kd = <8 nM

Humanized Antibody Binding Dissociation Constant

Table 4

| Hz1791 clone No. | Kd (nM) | C2B8 Kd (nM) |
|---|---|---|
| Aa008 | 11.68 | 6.61 |
| Aa012 | 9.96 | 4.68 |
| Fa007 | 11.34 | 5.63 |
| Fa008 | 8.83 | 4.91 |
| Sa023 | 14.66 | 6.13 |
| Va016 | 13.09 | 6.67 |
| Va024 | 7.50 | 6.47 |
| Af021 | 6.84 | 5.56 |
| Af025 | 8.67 | 4.29 |
| Ff019 | 8.50 | 5.70 |

(continued)

| Hz1791 clone No. | Kd (nM) | C2B8 Kd (nM) |
|---|---|---|
| Ff034 | 7.81 | 4.00 |
| Sf043 | 11.60 | 4.34 |
| Sf056 | 13.79 | 6.01 |
| Vf029 | 7.78 | 3.32 |
| Vf031 | 7.79 | 4.54 |
| As001 | 11.89 | 5.74 |
| As002 | 11.47 | 8.7 |
| Fs007 | 6.33 | 5.63 |
| Fs024 | 11.09 | 3.97 |
| Ss020 | 24.39 | 4.10 |
| Ss032 | 21.79 | 7.25 |
| Vs006 | 8.8 | 4.23 |
| Vs011 | 11.9 | 6.09 |
| Av004 | 10.71 | 5.76 |
| Av006 | 9.17 | 4.86 |
| Fv010 | 7.17 | 4.39 |
| Fv028 | 7.25 | 4.74 |
| Sv015 | 14.31 | 6.30 |
| Sv020 | 10.85 | 5.36 |
| Vv018 | 7.14 | 5.04 |
| Vv023 | 7.01 | 4.86 |

Humanized Antibody Binding Dissociation Constant

Table 5

| Group | Hz1791 clone | Kd (nM) |
|---|---|---|
| I (20 nM <) | ss | 23.09 |
| II (10 to 20 nM) | sa | 14.66 |
| | sf | 12.70 |
| | sv | 12.58 |
| | as | 11.68 |
| | aa | 10.82 |
| | vs | 10.35 |
| | va | 10.30 |
| | fa | 10.09 |
| III (8 to 10 nM) | av | 9.94 |
| | fs | 8.71 |
| | ff | 8.15 |

(continued)

| Group | Hz1791 clone | Kd (nM) |
|---|---|---|
| IV (< 8nM) | vf | 7.78 |
| | af | 7.76 |
| | fv | 7.21 |
| | vv | 7.07 |
| Control | c2B8 | $5.35 \pm 1.13$ |

The experimental results for apoptosis using 8 kinds of murine antibody are shown in Fig. 7a to Fig. 7d. We succeeded in dividing the 8 types of murine antibody and the known CD20 antibodies 2B8 and 2H7 into two types with respect to the four types of cell (omitting some exceptions).

   Group A: m0924, m1422, m1791, m2B8
   Group B: m1228, m1402 m1712, m1782, m2H7

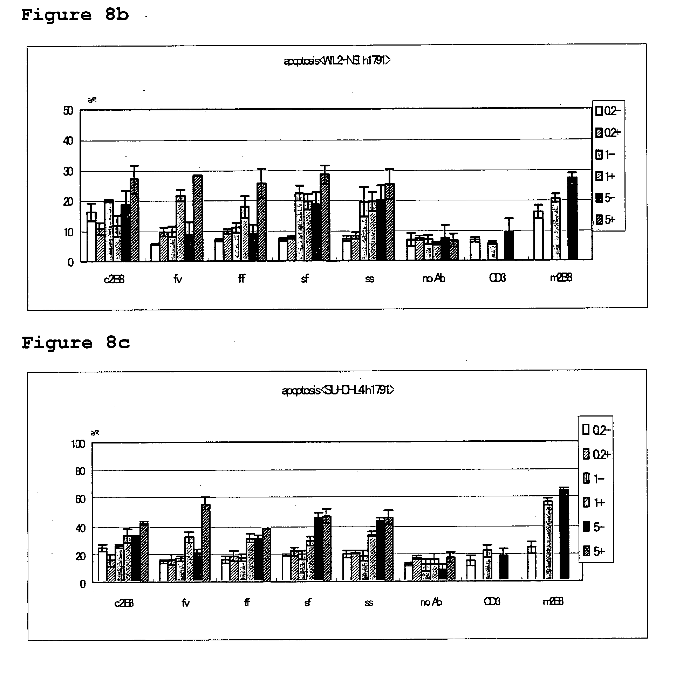

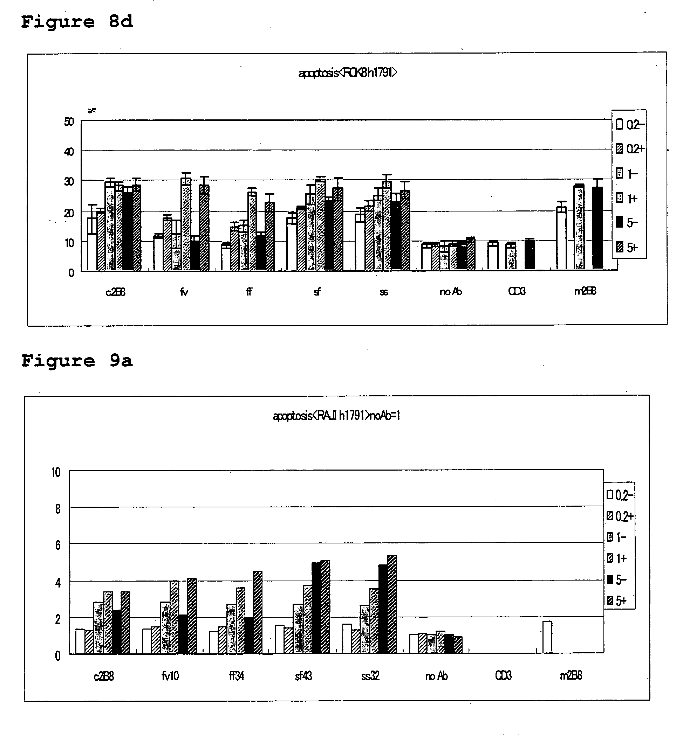

(However, m0924 is included in Group B with respect to SU-DHL4 cells).
In other words, anti CD20 antibodies belonging to Group A display sufficient capability to induce apoptosis independently and display approximately the same level of apoptosis inducing capability even under cross linking conditions with a secondary antibody. However Group B displays a large increase under cross linking conditions. Furthermore the affinity of antibodies belonging to Group A is equal to that of 2B8 antibody while the affinity of antibodies belonging to Group B display an affinity higher than that of 2B8. Consequently it can be inferred that Group A is more suitable for pharmaceutical use since Group A can induce apoptosis independently and does not require the presence of a secondary antibody to induce apoptosis.
Turning now to cell type, the ratio of apoptosis for RAJI, WIL2-NS, RCK8 has maximum values on the level of 30 to 40%. In contrast, SU-DHL4 displays a value of more than 80%.
The results for the four types of humanized antibodies are shown in Fig. 8a to Fig. 9d.
In the same manner as murine antibodies, the four types of humanized 1791 antibody are classified into two types with respect to the four types of cell.

   Group A: fv, ff
   Group B: sf, ss, C2B8

The antibodies fv, ff of Group A which have an equal affinity to that of C2B8 show almost no apoptosis activity under no cross linking condition and have clear activity under cross linking condition. The sf, ss antibodies of Group B display a greater dissociation constant and weaker affinity than the antibodies of Group A. These antibodies independently display apoptosis activity that is stronger than C2B8.
When anti CD20 antibodies independently display a sufficient capability of inducing apoptosis against B cells, the ratio under cross linking conditions is substantially the same. However when anti CD20 antibodies do not display a sufficient capability of inducing apoptosis due to antibody type or lack of affinity conditions, it is hypothesized that apoptosis activity will increase under cross linking conditions.

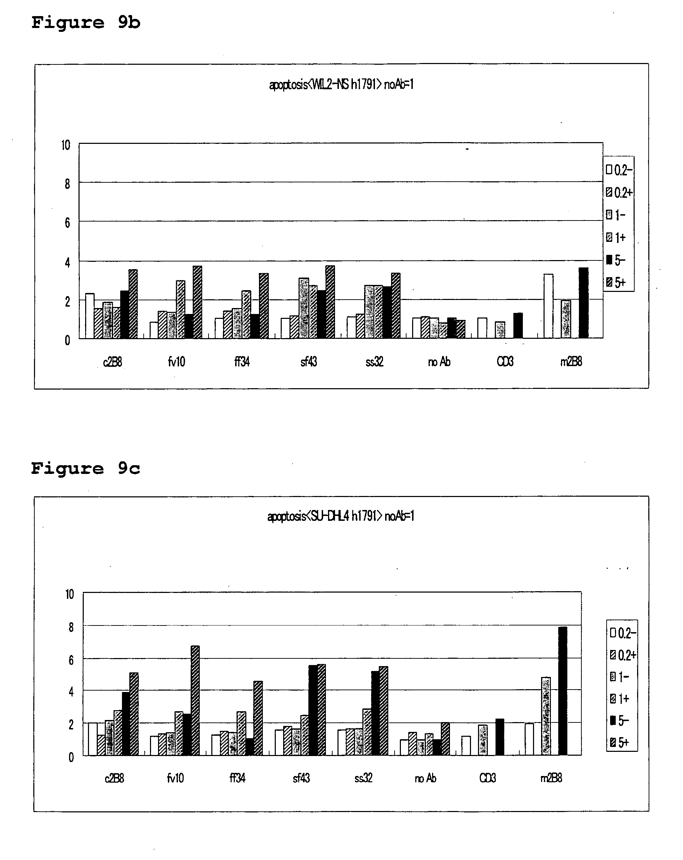

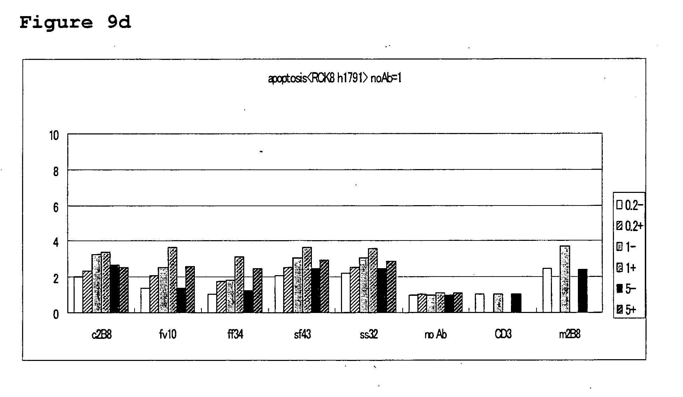

Fig. 9a to Fig. 9d are graphs showing early apoptosis (%) with a value of 1 when antibody is not added on the day of the experiment.

EXAMPLE 4

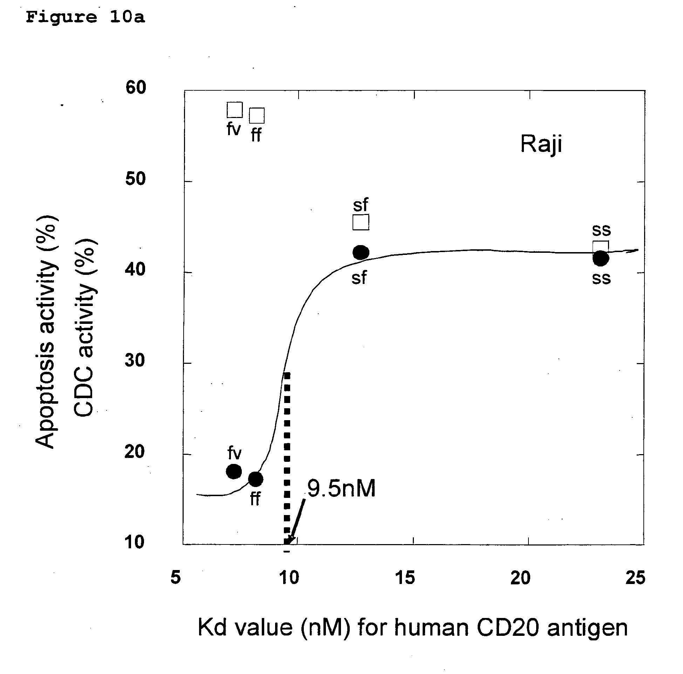

Relationship of Binding Dissociation Constant (Kd value) and Cell Growth Inhibiting Properties of Humanized Antibodies
On the basis of the division of humanized antibodies into Group A and Group B in Example 3, the relationship between Kd value (nM) with respect to human CD20 antigen, apoptosis inducing activity (5) and CDC activity (%) was examined with respect to the respective hz1791 clones shown in Table 5. The measurement of the Kd value was performed in the same manner as Example 2. The measurement of CDC activity was performed in the same manner as Example 2. (However in experiments examining the relationship between CDC activity and antibody amount, the antibodies were created in the manner described hereinafter). The measurement of ADCC was performed in the same manner as Example 2. The measurement of apoptosis activity was performed in the same manner as Example 3. B cells used in the experiments were Raji, SU-DHL4, WiL-2 and RCK8. Data regarding the four clones fv, ff, sf and ss for the respective cells are shown in Fig. 10a to Fig. 10d.

The same tendency was observed with respect to apoptosis activity for all cells used in the experiment. In other words, the higher the affinity (the smaller the Kd value) of an antibody, the lower the apoptosis inducing activity. The lower the affinity (the larger the Kd value) of an antibody, the higher the apoptosis inducing activity. Furthermore when the Kd value exceeds 13 nM, apoptosis activity tends to reach a constant value. Antibodies displaying high affinity do not display apoptosis activity independently and require cross linking using a secondary antibody in order to induce apoptosis. Antibodies displaying low affinity display apoptosis activity independently.

When using Raji and SU-DHL4 cells, CDC activity was observed to increase as the affinity (Kd value is small) increased. In contrast, when using WiL-2 and RCK8 cells, the Kd value displayed a tendency to reach an extremely high value near to 13 nM.

On the basis of the observations above, the following two selection criteria were determined to allow identification of candidate antibodies for use as pharmaceuticals:

Although the antibody does not induce apoptosis independently, affinity to CD20 antigen is extremely high and the antibody may display anti-cancer activity through CDC activity;

The antibody induces apoptosis independently, and from among the antibodies which may display anti-cancer activity through CDC activity and apoptosis, the antibody displays high affinity to CD20 antigen.

The former selection criterion is preferred when selecting antibodies which display high affinity to CD20 antigen and have CDC activity (a tendency for CDC activity to increase as the Kd value decreases). Since antibodies selected on this basis do not induce apoptosis independently, only the value for CDC activity is of concern. Clones satisfying the former selection criterion do not induce apoptosis in isolation, but have extremely high values for CDC activity and affinity and therefore promote cell deletion.

In the latter criterion, it is preferred to select antibodies in which the total of apoptosis and CDC activity of the antibody is high and which have high affinity to CD20 antigen. Thus although clones satisfying the latter criterion do not display high affinity, the antibodies have high apoptosis activity and therefore these antibodies promote cell deletion via the synergistic effect of inducing apoptosis and CDC.

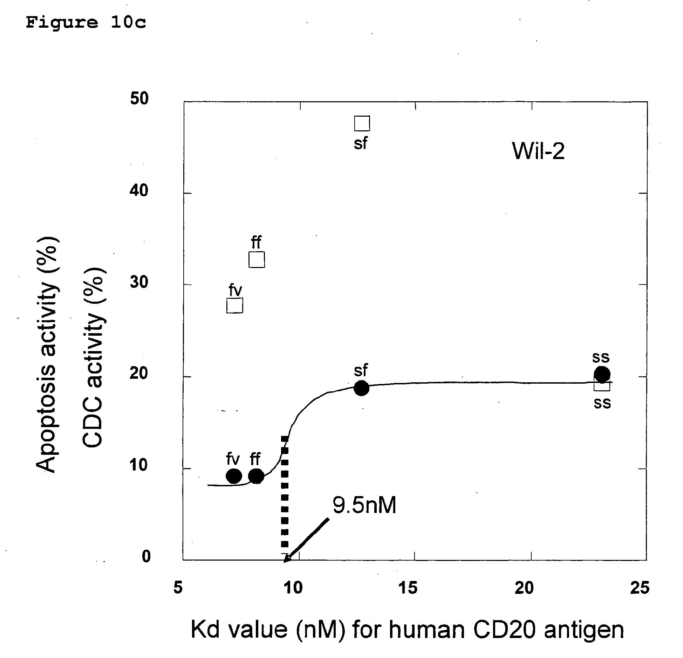

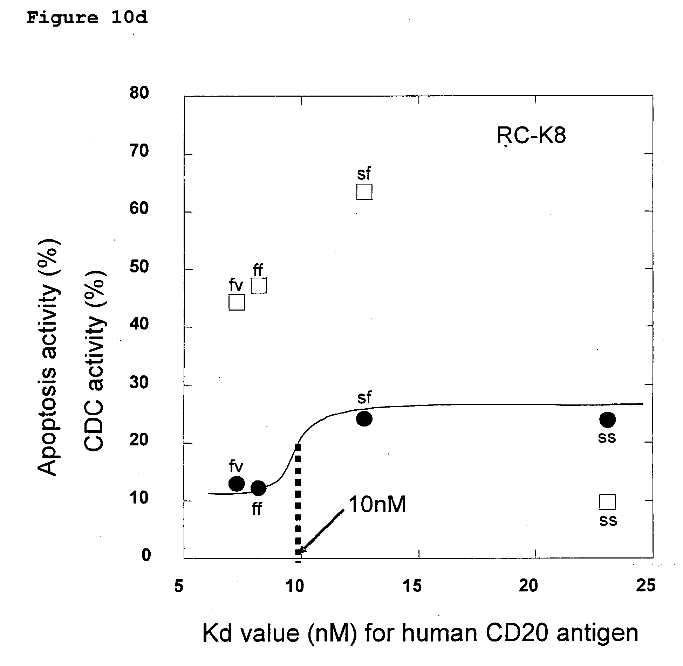

In order to determine boundary points for the two selection criteria above, Kd values giving the midpoint for apoptosis activity of the four cell types were represented graphically (the dotted line in the graphs in Fig. 10a to Fig. 10d). There is not a large difference in the Kd values giving the midpoint for apoptosis activity in these four graphs. The value for Raji cells is 9.5 nM, SU-DHL4 cells is 8.5 nM, WiL-2 cells is 9.5 nM and RCK8 cells is 10 nM. These results allowed the boundary point for the above two selection criteria to be set at a value of approximately 9.5 nM. The upper limit for the Kd value in the latter selection criteria was set at 13 nM being in a range wherein the total of apoptosis activity and CDC activity is as large as possible and moreover affinity is high (Kd value is small) taking into account the extremely high value for CDC activity seen in WiL2 cells and RCK8 cells (Fig. 10c and Fig. 10d).

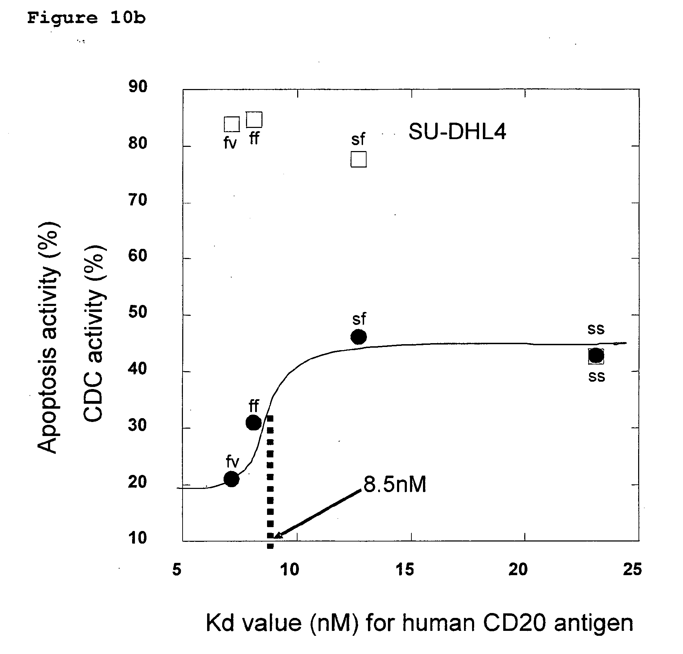

Thus according to the former selection criterion, the clones ff and fv are selected as antibodies which have a Kd value for human CD20 antigen of less than approximately 9.5 nM, have a Kd value as small as possible and high CDC activity (Fig. 10a and Fig. 10b). According to the latter selection criterion, the clone sf is selected as an antibody which has a Kd value for human CD20 antigen of approximately 9.5 nM to approximately 13 nM, has a total of apoptosis activity and CDC activity as high as possible and has a small Kd value (Fig. 10c and Fig. 10d).

CDC activity and apoptosis activity were measured by selecting fv, ff, sf and ss from the four types of humanized clones and using Raji cells, SU-DHL4 cell, WiL-2 cells and RCK8 cells. The results are shown in Table 6 and Table 7. Rituximab (C2B8) and the clone 1791 (c1791) were contrasted.

CDC Activity of Humanized Antibodies according to the Present Invention

Table 6

| Antibody | CDC (10 ug/ml) | | | |
| --- | --- | --- | --- | --- |
| | RAJI | WIL2NS | SUDHL4 | RC-K8 |
| | c2B8=100 | c2B8=100 | c2B8=100 | c2B8=100 |
| c2B8 | 100 | 100 | 100 | 3 |
| fv | 99 | 172 | 120 | 498 |
| ff | 98 | 203 | 121 | 530 |
| sf | 78 | 295 | 111 | 713 |

**EP 2 000 541 A1**

(continued)

| Antibody | CDC (10 ug/ml) | | | |
| --- | --- | --- | --- | --- |
| | RAJI | WIL2NS | SUDHL4 | RC-K8 |
| | c2B8=100 | c2B8=100 | c2B8=100 | c2B8=100 |
| ss | 73 | 120 | 61 | 108 |
| c1791 | 99 | 529 | 119 | 100 |

The results in Table 6 show that the clones ff and fv display a CDC activity which is equal to or stronger than the activity of rituximab. Furthermore sf shows extremely strong CDC activity with respect to WiL2 and RCK8 cells.

An experiment was conducted to investigate the relationship between CDC activity and antibody concentration. The antibodies used were more highly purified than the antibodies treated according to Example 2. Purification was carried as described hereinafter.

Antibody producing cell strains (genetically recombinant CHO-DG44 cells) were cultured in IS CHO-CD/w medium (Irvine Scientific, Cat. No. 91119) containing Hydrolysate supplemented with 4 nM GlutaMax (Invitrogen, Cat 35050-061) and 200 $\mu$g/ml of G418 (Sigma, Cat. No.

A1720-5G) in a $CO_2$ incubator under an atmosphere of 5% $CO_2$ at 37°C. The cells were passaged twice per week. Cell culture solution approximately two weeks after subculturing was centrifuged at 3,500 rpm for five minutes at room temperature. The supernatant was recovered, filtered using a 0.45 $\mu$m syringe filter and equilibrated using 50 nM Tris-HCl, pH 7.0. After adding culture medium supernatant to a Hi Trap Protein A HP column (GE Healthcare, Cat No. 17-0402-01), washing was performed using 50 nM Tris-HCl, pH 7.0. Elutions were obtained using 0.1 M citric acid pH 4.0. 400 $\mu$l was collected on each occasion and neutralized with 40 $\mu$l (or a 10/1 amount) of 1 M Tris-HCl, pH 9.0. After dialyzing twice against a 100 times amount of PBS for 2.5 hours using a Slyde-A-Lyzer 10K Dialysis Cassetes (PIERCE Cat No. 66453), dialysis was performed for 15 to 18 hours on one occasion.

After dialysis, the sample was concentrated using a VIVASPIN 50,000 MWCO PES (VIVASCIENCE Cat No. VS0231). The sample was added to a HiLoad 16/60 superdex 200 prep grade column (GE Healthcare Cat No. 17-1069-01) equilibrated with PBS. The sample was filtered using a 0.22 $\mu$m syringe filter and concentrated using a VIVASPIN 50,000 MWCO PES (VIVASCIENCE Cat No. VS0231). The antibody concentration was calculated from an A280 value using a BECKMAN COULTER DU530.

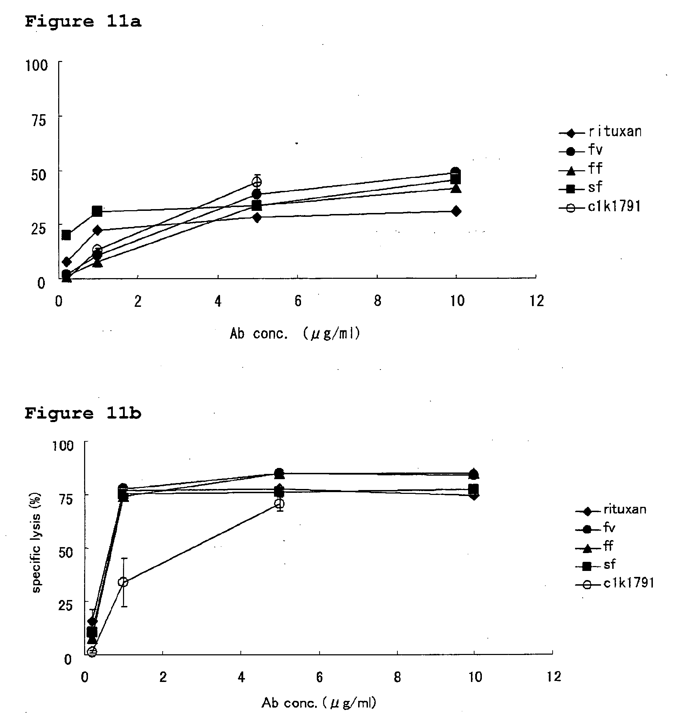

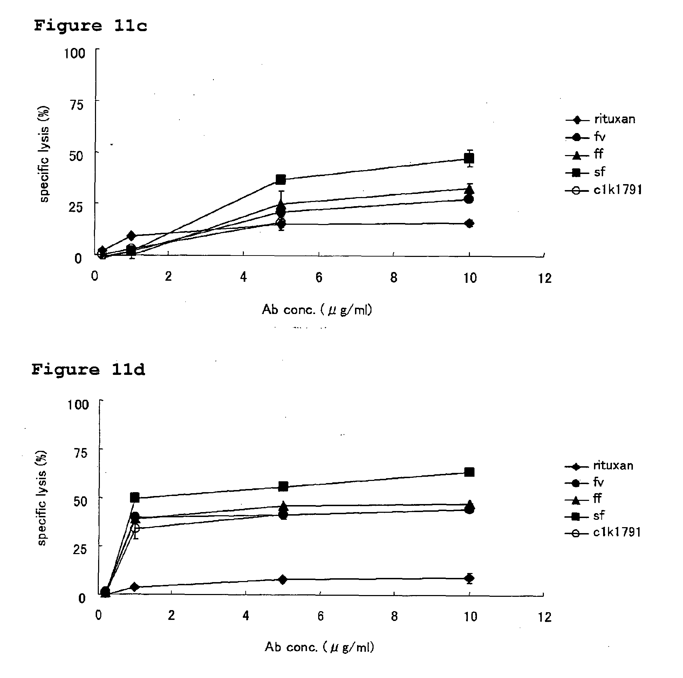

The relationship between concentration and CDC activity of the chimera antibody c1k179 and the humanized antibodies fv, ff, sf, ss against Raji cells, SU-DHL4 cells, WiL-2 cells and RCK8 cells is shown in Fig. 11a to Fig. 11d. In all series of experiments, the CDC activity of the humanized antibodies fv, ff, sf, ss at a concentration of 5 $\mu$g/ml or more was equal to or greater than the activity of rituximab (C2B8).

The clones fv and ff (clones expected to display CDC activity) were selected as having a Kd value for Raji cells (floating cells) of less than 9.5 nM and moreover as being antibodies having high CDC activity. These clones displayed higher CDC activity than rituximab with respect to all cell types at a concentration of 5 $\mu$g/ml or more. CDC activity with respect to SU-DHL4 cells was particularly high and CDC activity was also high with respect to Raji cells and RCK8 cells.

The clone sf (both CDC activity and apoptosis expected) was selected as an antibody which has a Kd value for Raji cells (floating cells) of approximately 9.5 nM to approximately 13 nM, has a total of apoptosis activity and CDC activity as high as possible and has a small Kd value. This clone displayed cell lytic activity which was equal to or higher than rituximab with respect to all cell types at a concentration of 5 $\mu$g/ml or more. Cell lytic activity with respect to SU-DHL4 cells was particularly high and cell lytic activity was also high with respect to WiL2 cells and RCK8 cells.

Apoptosis activity of fv, ff, sf, and ss against Raji cells, SU-DHL4 cells, WiL-2 cells and RCK8 cells was examined and the results are shown in Table 7.

Apoptosis Activity of Humanized Antibodies according to the Present Invention

Table 7

| Antibody | Affinity (average) | Apoptosis (5 ug/ml) | | | | | | | |
|---|---|---|---|---|---|---|---|---|---|
| | Raji | RAJI | | Wi12-NS | | DHL4 | | RCK8 | |
| | Kd (nM) | m2B8=100 | XL w/wo | m2B8=100 | XL w/wo | m2B8=100 | XL w/wo | m2B8=100 | XL w/wo |
| c2B8 | 5.35 | 100 | 1.4 | 100 | 1.4 | 100 | 1.3 | 100 | 0.9 |
| fv | 7.21 | 88 | 1.9 | 49 | 3.1 | 65 | 2.7 | 50 | 1.9 |
| ff | 8.15 | 84 | 2.2 | 49 | 2.8 | 96 | 1.2 | 47 | 1.9 |
| sf | 12.70 | 205 | 1.0 | 100 | 1.5 | 143 | 1.0 | 93 | 1.2 |
| ss | 23.09 | 202 | 1.1 | 108 | 1.3 | 133 | 1.1 | 92 | 1.2 |
| no Ab | | 42 | 0.9 | 41 | 0.9 | 26 | 2.0 | 37 | 1.1 |

From the results in Table 7, the clone sf has apoptosis inducing activity which is equal to or stronger than rituximab. Sufficient apoptosis activity was induced independently without the need for a secondary antibody. Furthermore the sum of CDC activity and apoptosis activity of the clone sf exceeded that of rituximab with respect to all cells used in the experiment. In particular, a high value was observed with respect to WiL2 cells and SU-DHL4 cells (Table 6, Fig. 11a to Fig. 11d and Table 7).

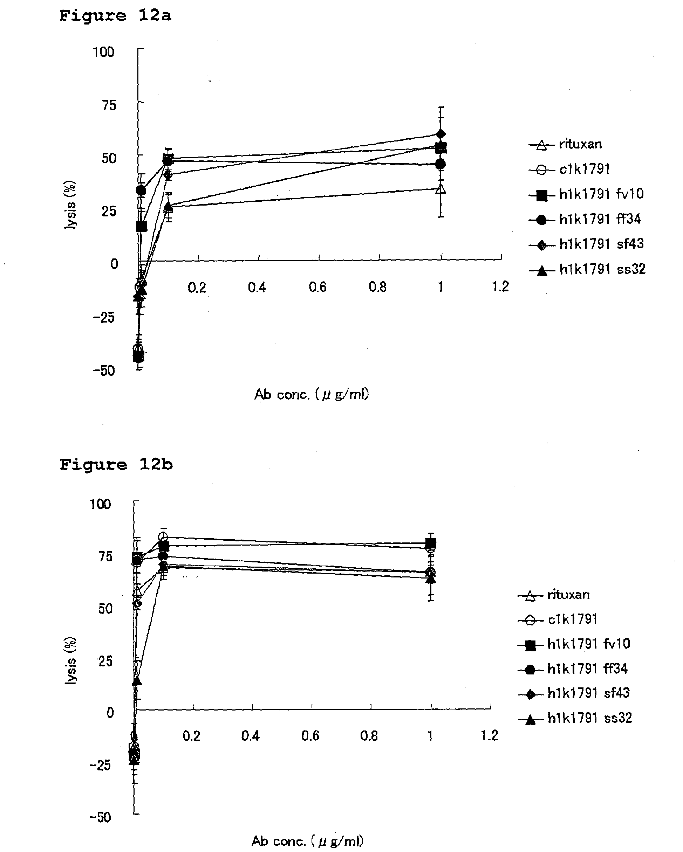

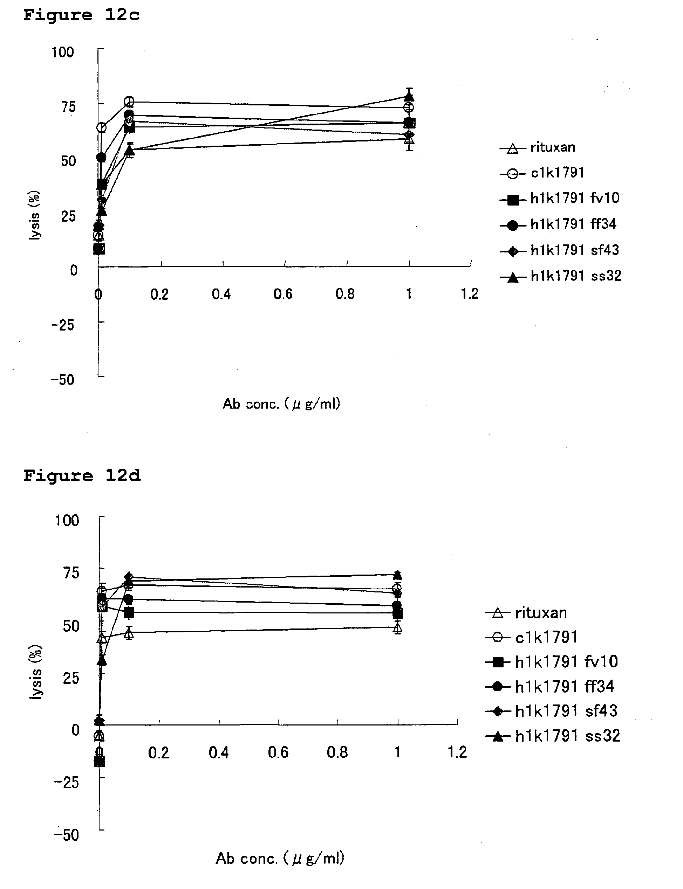

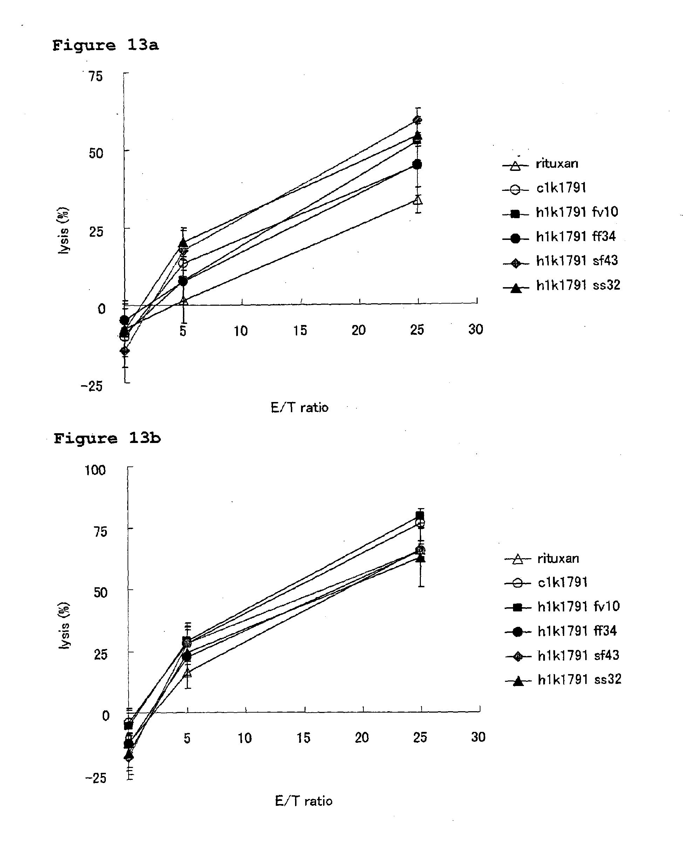

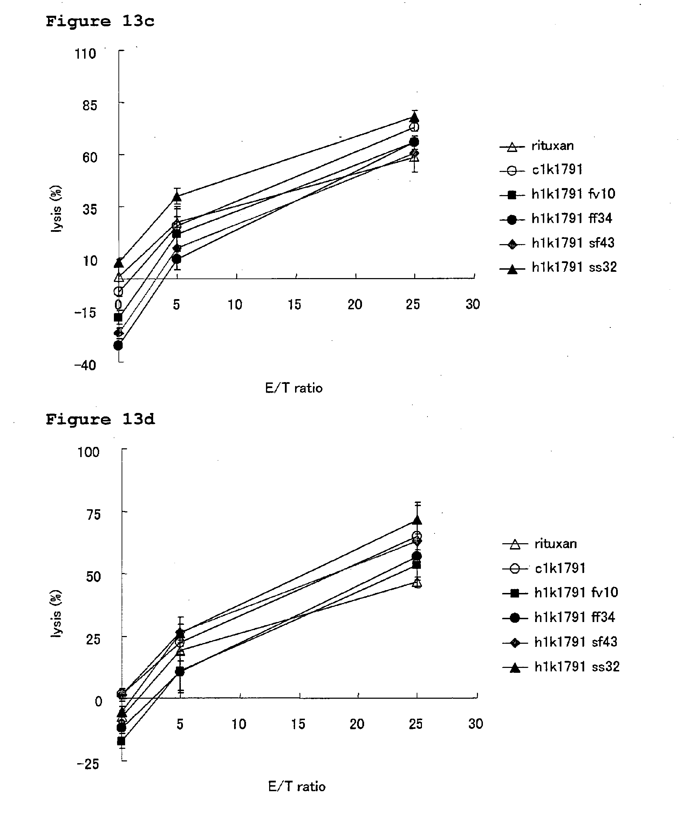

The ADCC activity of fv, ff, sf, and ss against Raji cells, SU-DHL4 cells, WiL-2 cells and RCK8 cells was examined. The relationship between antibody concentration and ADCC is shown in Fig. 12a to Fig. 12d (E:T ratio is 25). The relationship between E:T ratio and ADCC is shown in Fig. 13a to Fig. 13d (antibody concentration is 1 μg/ml). The ADCC activity of fv, ff, sf and ss in all experiments was equal to or greater than the activity of rituximab (C2B8). The results for ADCC activity also demonstrate the efficacy fv, ff, sf and ss selected in this experiment.

These results show that humanized monoclonal antibodies according to the present invention selected in the manner described above display higher cell lytic activity than the cell lytic activity of rituximab. Thus humanized antibodies selected according to the selection criteria of the present invention are thought to have therapeutic efficacy in B cell mediated diseases allowing for their use as pharmaceuticals.

INDUSTRIAL APPLICABILITY

According to the present invention, a humanized anti human CD20 monoclonal antibody and a method of selection therefor is provided, the antibody displaying biological activity suitable for use as a pharmaceutical and high binding affinity to natural human CD20 molecules. These antibodies have therapeutic efficacy with respect to B cell mediated diseases allowing for their use as pharmaceuticals.

SEQ. ID. NO.: 17: L chain V region sequence of humanized antibody abb 1791
SEQ. ID. NO.: 18: L chain V region sequence of humanized antibody fra 1791
SEQ. ID. NO.: 19: L chain V region sequence of humanized antibody sdr 1791
SEQ. ID. NO.: 20: L chain V region sequence of humanized antibody Ven 1791
SEQ. ID. NO.: 21: H chain V region sequence of humanized antibody abb 1791
SEQ. ID. NO.: 22: H chain V region sequence of humanized antibody fra 1791
SEQ. ID. NO.: 23: H chain V region sequence of humanized antibody sdr 1791
SEQ. ID. NO.: 24: H chain V region sequence of humanized antibody Ven 1791
SEQ. ID. NO.: 25: primer
SEQ. ID. NO.: 26: primer

**Claims**

1. A humanized anti CD20 monoclonal antibody which has growth inhibiting activity on cells containing human CD20 antigen, the immunogen being a human B cell strain expressing human CD20 antigen and a cell strain transformed with human CD20 DNA which is a non-human cell and derived from an animal which is different from the animal to be immunized, and which meets the selection criteria below:

   (i) the antibody having a dissociation constant (Kd value) for human CD20 antigen of less than approximately 9.5 nM and CDC activity on B cells equal to or greater than that of 2B8 antibody.

2. A humanized anti CD20 monoclonal antibody which has growth inhibiting activity on cells containing human CD20 antigen, the immunogen being a human B cell strain expressing human CD20 antigen and a cell strain transformed with human CD20 DNA which is a non-human cell and derived from an animal which is different from the animal to be immunized, and which meets the selection criteria below:

   (a) the antibody having a dissociation constant (Kd value) for human CD20 antigen of less than approximately 9.5 nM and CDC activity on Raji cells (suspended cells) or SU-DHL4 cells equal to or greater than that of 2B8 antibody.

3. A humanized anti CD20 monoclonal antibody which has growth inhibiting activity on cells containing human CD20 antigen, the immunogen being a human B cell strain expressing human CD20 antigen and a cell strain transformed with human CD20 DNA which is a non-human cell and derived from an animal which is different from the animal to be immunized, and which meets the selection criteria below:

   (ii) the antibody having a Kd value for human CD20 antigen in the range of from approximately 9.5 nM to approximately 13 nM and a total of apoptosis activity and CDC activity on B cells equal to or greater than that of 2B8 antibody.

4. A humanized anti CD20 monoclonal antibody which has growth inhibiting activity on cells containing human CD20 antigen, the immunogen being a human B cell strain expressing human CD20 antigen and a cell strain transformed with human CD20 DNA which is a non-human cell and derived from an animal which is different from the animal to be immunized, and which meets the selection criteria below:

   (b) the antibody having a Kd value for human CD20 antigen in the range of from approximately 9.5 nM to approximately 13 nM and a total of apoptosis activity and CDC activity on WiL2 cells or RCK8 cells equal to or greater than that of 2B8 antibody.

5. The humanized anti CD20 monoclonal antibody according to claim 1 or 2, comprising a combination of the L chain set forth in SEQ ID No: 18 and the H chain set forth in SEQ ID No: 22.

**6.** The humanized anti CD20 monoclonal antibody according to claim 1 or 2, comprising a combination of the L chain set forth in SEQ ID No: 18 and the H chain set forth in SEQ ID No: 24.

**7.** The humanized anti CD20 monoclonal antibody according to claim 3 or 4, comprising a combination of the L chain set forth in SEQ ID No: 19 and the H chain set forth in SEQ ID No: 22.

**8.** A therapeutic agent for the treatment of B cell mediated diseases, comprising as an active ingredient the humanized anti CD20 monoclonal antibody according to any one of claims 1 to 7.

## Figure 1

pNOW-Ab

Figure 2

pNOW

Figure 3a

Figure 3b

## Figure 3c

Day2 (2ug/ml)

## Figure 3d

Day2 (4ug/ml)

Figure 4a

**Figure 4b**

## Figure 4c

## Figure 4d

## Figure 5a

Figure 5b

WiL-2  5.0 $\mu$g/ml

**Figure 5c**

SU-DHL4   5.0 $\mu$ g/ml

# Figure 5d

RC–K8    5.0 μg/ml

# Figure 6a

## Figure 6b

WiL2 CDC

Legend:
- riuxan
- 1k0924
- 1k1402
- 1k1422
- 1k1712
- 1k1736
- 1k1791

Y-axis: lysis /%
X-axis: Ab conc. / $\mu$ g/ml

## Figure 6c

## Figure 6d

## Figure 7a

## Figure 7b

## Figure 7c

## Figure 7d

## Figure 8a

Figure 8b

Figure 8c

## Figure 8d

apoptosis<ROK8h1791>

## Figure 9a

apoptosis<RAJI h1791>noAb=1

**Figure 9b**

apoptosis<WIL2-NS h1791> noAb=1

**Figure 9c**

apoptosis<SU-DHL4 h1791> noAb=1

## Figure 9d

apoptosis⟨RCK8 h1791⟩ noAb=1

Figure 10a

Kd value (nM) for human CD20 antigen

Figure 10b

Figure 10c

**Figure 10d**

## Figure 11a

Ab conc. (μg/ml)

Legend:
- ━◆━ rituxan
- ━●━ fv
- ━▲━ ff
- ━■━ sf
- ━○━ c1k1791

## Figure 11b

specific lysis (%)

Ab conc. (μg/ml)

Legend:
- ━◆━ rituxan
- ━●━ fv
- ━▲━ ff
- ━■━ sf
- ━○━ c1k1791

## Figure 11c

## Figure 11d

**Figure 12a**

Ab conc. ($\mu$g/ml)

Legend:
- rituxan
- c1k1791
- h1k1791 fv10
- h1k1791 ff34
- h1k1791 sf43
- h1k1791 ss32

**Figure 12b**

Ab conc. ($\mu$g/ml)

Legend:
- rituxan
- c1k1791
- h1k1791 fv10
- h1k1791 ff34
- h1k1791 sf43
- h1k1791 ss32

## Figure 12c

Legend:
- rituxan
- c1k1791
- h1k1791 fv10
- h1k1791 ff34
- h1k1791 sf43
- h1k1791 ss32

y-axis: lysis (%)
x-axis: Ab conc. ($\mu$ g/ml)

## Figure 12d

Legend:
- rituxan
- c1k1791
- h1k1791 fv10
- h1k1791 ff34
- h1k1791 sf43
- h1k1791 ss32

y-axis: lysis (%)
x-axis: Ab conc. ($\mu$ g/ml)

**Figure 13a**

**Figure 13b**

## Figure 13c

E/T ratio

- rituxan
- c1k1791
- h1k1791 fv10
- h1k1791 ff34
- h1k1791 sf43
- h1k1791 ss32

## Figure 13d

E/T ratio

- rituxan
- c1k1791
- h1k1791 fv10
- h1k1791 ff34
- h1k1791 sf43
- h1k1791 ss32

## INTERNATIONAL SEARCH REPORT

| International application No. |
| --- |
| PCT/JP2006/313499 |

A.  CLASSIFICATION OF SUBJECT MATTER
*C12P21/08*(2006.01)i, *A61K39/395*(2006.01)i, *C07K16/28*(2006.01)i, *C12N15/00* (2006.01)i

According to International Patent Classification (IPC) or to both national classification and IPC

B.  FIELDS SEARCHED

Minimum documentation searched (classification system followed by classification symbols)
C12P21/08, A61K39/395, C07K16/28, C12N15/00

Documentation searched other than minimum documentation to the extent that such documents are included in the fields searched

Electronic data base consulted during the international search (name of data base and, where practicable, search terms used)
BIOSIS/MEDLINE/WPIDS(STN), JMEDPlus/JST7580/JSTPlus(JDream2), GenBank/EMBL/DDBJ/GeneSeq, SwissProt/Geneseq

C.  DOCUMENTS CONSIDERED TO BE RELEVANT

| Category* | Citation of document, with indication, where appropriate, of the relevant passages | Relevant to claim No. |
| --- | --- | --- |
| X/Y | STEIN R. et al.,'Characterization of a new humanized anti-CD20 monoclonal antibody, IMMU-106, and its use in combination with the humanized anti-CD22 antibody, epratuzumab, for the therapy of non-Hodgkin's lymphoma.', Clin. Cancer Res. (2004) vol. 10, pp. 2868-2878, particularly, page 2870, description in the 2nd paragraph of upper right column; Figs. 3, 6 | 1,2/3-8 |
| Y | TEELING J.L. et al.,'Characterization of new human CD20 monoclonal antibodies with potent cytolytic activity against non-Hodgkin lymphomas.', Blood (2004) vol. 104, pp. 1793-1800, particularly, Fig. 4 | 3-8 |

|X| Further documents are listed in the continuation of Box C.          | | See patent family annex.

| * | Special categories of cited documents: |
| --- | --- |
| "A" | document defining the general state of the art which is not considered   to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| 20 November, 0007 (20.11.07) | 12 December, 2006 (12.12.06) |

| Name and mailing address of the ISA/ | Authorized officer |
| --- | --- |
| Japanese Patent Office | |
| Facsimile No. | Telephone No. |

Form PCT/ISA/210 (second sheet) (April 2005)

**INTERNATIONAL SEARCH REPORT**

| | International application No. |
|---|---|
| | PCT/JP2006/313499 |

C (Continuation).   DOCUMENTS CONSIDERED TO BE RELEVANT

| Category* | Citation of document, with indication, where appropriate, of the relevant passages | Relevant to claim No. |
|---|---|---|
| Y | LEONARD J.P., 'Targeting CD 20 in follicular NHL: novel anti-CD20therapies, antibody engineering, and the use of radioimmunoconjugates.', Hematology Am. Soc. Hematol. Educ. Program (2005) pp. 335-339, particularly, page 336, left column, 2nd paragraph to same page, right column, 2nd paragraph; table 1 | 3-8 |
| Y | CRAGG, M.S. et al., 'Complement-mediated lysis by anti-CD20 mAb correlates with segregation into lipid rafts.', Blood (2003) vol. 101, pp. 1045-1052, particularly, table 1 | 3-8 |
| A | CARTRON G. et al., 'Therapeutic activity of humanized anti-CD20 monoclonal antibody and polymorphism in IgG Fc receptor FcγRIIIa gene.', Blood (2002) vol. 99, pp. 754-758 | 1-8 |
| A | CLYNES R.A., 'Inhibitory Fc receptors modulate *in vivo* cytoxicity against tumor targets.', Nature Med., (2000) vol. 6, pp. 443-446 | 1-8 |
| A | BYRD J.C., 'The mechanism of tumor cell clearance by rituximab in vivo in patients with B-cell chronic lymphocytic leukemia: evidence of caspase activation and apoptosis induction.', Blood (2002) vol. 99, pp. 1038-1043 | 1-8 |

Form PCT/ISA/210 (continuation of second sheet) (April 2005)

**EP 2 000 541 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9411026 A
- US 5736137 A
- JP 3582965 B

**Non-patent literature cited in the description**

- **COIFFIER B et al.** *Blood,* 1998, vol. 92, 1297-32
- **EDWARD JC et al.** *Rheumatology (Oxford,* 2001, vol. 40, 205-11
- **ZAJA F et al.** *Heamatologica,* 2002, vol. 87, 189-95
- **PERROTTA S et al.** *Br J Haematol,* 2002, vol. 116, 465-7
- **REFF et al.** *Blood,* 1994, vol. 83, 435-445
- **MALONEY et al.** *Blood,* 1996, vol. 88, 637a
- *IDEC Pharmaceuticals Corporation News Release,* 08 December 1998
- **MITCHELL ER et al.** *Blood,* 1994, vol. 82, 435-445
- **MIYAMOTO T ; MIN W ; LILLEHOJ HS.** *Avian Dis.,* January 2002, vol. 46 (1), 10-6
- **ISHIDA T ; IMAI K.** *Nippon Rinsho,* March 2003, vol. 60 (3), 439-444
- **ISHIDA T ; IMAI K.** *Nippon Rinsho,* March 2002, vol. 60 (3), 439-444
- **EDUARDO A.PADLAN.** *Molecular Immunology,* 1991, vol. 28 (4/5), 489-498
- **EDUARDO A. PADLAN.** *The FASEB Journal,* vol. 9, 133-139
- **TAI TE WU ; ELVIN A. KABAT.** *Molecular Immunology,* 1992, vol. 29 (9), 1141-1146
- **MONOKURONARU KOTAI ; SEIKAGAKU JIKKENHO.** Monoclonal Antibody, Biochemical Experiment Method. Toshiaki OSAWA, 1989
- **OI, V.T. ; L.A. HERZENBERG.** Selected Methods in Cellular Immunology. Freeman and Co, 1980, 351
- New Cultured Cell Experimental Methods in Molecular Biology Research. **SCATCHARD, G.** Ann. N.Y. Acad. Sci. Yodosha Co., Ltd, 1949, vol. 51, 660-672
- BioManual. 212-217

93

SEQUENCE LISTING

<110> OSAKA UNIVERSITY

<120> Humanized anti-CD20 monoclonal antibody

<130> 38-110

<140> EP 06 767 958.9
<141> 2006-07-06

<150> PCT/JP2006/304370
<151> 2006-03-07

<160> 26

<170> PatentIn version 3.2

<210> 1
<211> 106
<212> PRT
<213> Mus musculus

<400> 1

Gln Ile Val Leu Thr Gln Ser Pro Pro Ile Met Ser Ala Ser Leu Gly
1               5                   10                  15

Glu Glu Ile Thr Leu Thr Cys Ser Ala Ser Ser Arg Val Ser Tyr Met
                20                  25                  30

Leu Trp Tyr Gln Gln Lys Ser Gly Thr Ser Pro Lys Leu Leu Ile Tyr
            35                  40                  45

Ser Thr Ser Asn Leu Ala Ser Gly Val Pro Ser Arg Phe Ser Gly Ser
        50                  55                  60

Gly Ser Gly Thr Phe Tyr Ser Leu Thr Ile Ser Ser Val Glu Ala Glu
65                  70                  75                  80

Asp Ala Ala Asp Tyr Tyr Cys His Gln Trp Thr Ser Asn Pro Cys Thr
                85                  90                  95

Phe Gly Gly Gly Thr Lys Leu Glu Ile Lys
                100                 105

<210> 2
<211> 107
<212> PRT
<213> Mus musculus

<400> 2

Ser Thr Val Met Thr Gln Thr Pro Lys Phe Leu Leu Val Ser Ala Gly
1               5                   10                  15

Asp Arg Val Thr Ile Thr Cys Lys Ala Ser Gln Ser Val Ser Asn Asp
                20                  25                  30

Val Ala Trp Tyr Gln Gln Lys Pro Gly Gln Ser Pro Lys Val Leu Ile
35                    40                 45

Tyr Phe Ala Ser Asn Arg Tyr Thr Gly Val Pro Asp Arg Phe Thr Gly
50                    55                 60

Ser Gly Tyr Gly Thr Asp Phe Thr Phe Thr Ile Asn Thr Val Gln Ala
65                    70                 75                 80

Glu Asp Leu Ala Val Tyr Phe Cys Gln Gln Asp Tyr Ser Ser Pro Leu
85                    90                 95

Thr Phe Gly Ala Gly Thr Lys Leu Glu Leu Lys
100                   105

<210>   3
<211>   106
<212>   PRT
<213>   Mus musculus

<400>   3

Gln Ile Val Leu Ser Gln Ser Pro Ala Ile Leu Ser Ala Ser Pro Gly
1                5                  10                 15

Glu Lys Val Thr Met Thr Cys Arg Ala Ser Ser Ser Val Ser Tyr Met
20                   25                 30

His Trp Tyr Gln Gln Arg Pro Gly Ser Ser Pro Lys Pro Trp Ile Tyr
35                    40                 45

Ala Thr Ser Asn Leu Ala Ser Gly Val Pro Ala Arg Phe Ser Gly Ser
50                    55                 60

Gly Ser Gly Thr Ser Tyr Tyr Phe Thr Ile Ser Arg Val Glu Ala Glu
65                    70                 75                 80

Asp Ala Ala Thr Tyr Tyr Cys Gln Gln Trp Asn Ser Asn Pro Pro Thr
85                    90                 95

His Gly Gly Gly Thr Lys Leu Glu Ile Lys
100                   105

<210>   4
<211>   106
<212>   PRT
<213>   Mus musculus

<400>   4

Gln Ile Val Leu Ser Gln Ser Pro Ala Ile Leu Ser Ala Ser Pro Gly
1                5                  10                 15

Glu Lys Val Thr Met Thr Cys Arg Ala Ser Ser Ser Val Ser Tyr Met
20                   25                 30

```
Asp Trp Tyr Gln Gln Lys Pro Gly Ser Ser Pro Lys Pro Trp Ile Tyr
        35              40          45

Ala Thr Ser Asn Leu Ala Ser Gly Val Pro Ala Arg Phe Ser Gly Ser
    50              55              60

Gly Ser Gly Thr Ser Tyr Ser Leu Thr Ile Ser Arg Val Glu Ala Glu
65              70              75              80

Asp Thr Ala Thr Tyr Tyr Cys Gln Gln Trp Thr Phe Asn Pro Pro Thr
            85              90              95

Phe Gly Ser Gly Thr Lys Leu Glu Ile Lys
            100             105
```

<210> 5
<211> 106
<212> PRT
<213> Mus musculus

<400> 5

```
Gln Ile Val Leu Ser Gln Ser Pro Ala Ile Leu Ser Ala Ser Pro Gly
1               5               10              15

Glu Lys Val Thr Met Thr Cys Arg Ala Ser Ser Ser Val Ser Tyr Met
            20              25              30

His Trp Tyr Gln Gln Lys Pro Gly Ser Ser Pro Lys Pro Trp Ile Tyr
        35              40          45

Ala Thr Ser Asn Leu Ala Ser Gly Val Pro Ala Arg Phe Ser Gly Ser
    50              55              60

Gly Ser Gly Thr Ser Tyr Ser Leu Thr Ile Thr Arg Val Glu Ala Glu
65              70              75              80

Asp Ala Ala Thr Tyr Tyr Cys Gln Gln Trp Thr Phe Asn Pro Pro Thr
            85              90              95

Phe Gly Ala Gly Thr Lys Leu Glu Leu Lys
            100             105
```

<210> 6
<211> 106
<212> PRT
<213> Mus musculus

<400> 6

```
Gln Ile Val Leu Ser Gln Ser Pro Ala Ile Leu Ser Ser Ser Pro Gly
1               5               10              15

Glu Lys Val Thr Met Thr Cys Arg Ala Ser Ser Ser Val Ser Tyr Met
```

```
                    20                    25                    30
Leu Trp Tyr Gln Gln Lys Pro Gly Ser Ser Pro Glu Pro Trp Ile Tyr
        35                40                45
Ala Thr Ser Asn Leu Ala Ser Gly Val Pro Ala Arg Phe Ser Gly Gly
        50                55                60
Gly Ser Gly Thr Ser Tyr Ser Leu Thr Ile Ser Arg Val Glu Ala Glu
65                70                75                80
Asp Ala Ala Thr Tyr Tyr Cys Gln Gln Trp Thr Phe Asn Pro Pro Thr
            85                90                95
Phe Gly Gly Gly Thr Lys Leu Glu Ile Lys
            100               105
```

```
<210>   7
<211>   107
<212>   PRT
<213>   Mus musculus

<400>   7
```

```
Asp Ile Leu Leu Thr Gln Ser Pro Ala Ile Leu Phe Val Ser Pro Gly
1               5                10                15

Glu Arg Val Ser Leu Ser Cys Arg Ala Ser Gln Asn Ile Gly Thr Ser
            20                25                30
Ile His Trp Tyr Gln Gln Arg Thr Asn Gly Ser Pro Arg Leu Leu Ile
        35                40                45
Lys Tyr Ala Ser Glu Ser Phe Ser Gly Ile Pro Ser Arg Phe Ser Gly
    50                55                60
Ser Gly Ser Gly Thr Asp Phe Thr Leu Ser Ile Asn Ser Val Glu Ser
65                70                75                80
Glu Asp Ile Ala Asp Tyr Tyr Cys Gln Gln Ser Asn Ser Trp Pro Phe
            85                90                95
Thr Phe Gly Ser Gly Thr Lys Leu Glu Ile Lys
            100               105
```

```
<210>   8
<211>   108
<212>   PRT
<213>   Mus musculus

<400>   8
```

```
Glu Ile Ile Leu Thr Gln Ser Pro Thr Thr Met Ala Ala Ser Pro Gly
1               5                10                15

Glu Lys Ile Thr Ile Thr Cys Ser Ala Ser Ser Ser Ile Ser Ser Tyr
            20                25                30
```

```
Tyr Leu Arg Trp Tyr Gln Gln Lys Pro Gly Phe Ser Pro Lys Val Leu
        35              40              45

Ile Tyr Arg Thr Ser Asn Leu Ala Ser Gly Val Pro Ala Arg Phe Ser
    50              55              60

Gly Ser Gly Ser Gly Thr Ser Tyr Ser Leu Thr Ile Gly Thr Met Glu
65              70              75              80

Ala Glu Asp Val Ala Thr Tyr Tyr Cys Gln Gln Gly Asn Thr Val Pro
            85              90              95

Leu Thr Phe Gly Ser Gly Thr Lys Leu Glu Ile Lys
            100             105
```

```
<210>  9
<211>  123
<212>  PRT
<213>  Mus musculus

<400>  9
```

```
Gln Val Gln Leu Gln Gln Pro Gly Ala Glu Leu Val Lys Pro Gly Ala
1               5               10              15

Ser Val Lys Met Ser Cys Arg Ala Ser Gly Tyr Thr Phe Thr Asn Tyr
            20              25              30

Asn Met His Trp Ile Lys Gln Thr Pro Gly Gln Gly Leu Glu Trp Ile
        35              40              45

Gly Ala Ile Tyr Pro Gly Ser Gly Asp Thr Ser Tyr Asn Arg Lys Phe
    50              55              60

Lys Gly Lys Ala Thr Leu Thr Ala Asp Thr Ser Ser Ser Thr Ala Tyr
65              70              75              80

Met Gln Phe Ser Ser Leu Thr Ser Ala Asp Ser Ala Val Tyr Tyr Cys
            85              90              95

Ala Arg Phe Thr Tyr Tyr Tyr Gly Gly Thr Tyr Gly Ala Met Asp Tyr
            100             105             110

Trp Gly Gln Gly Thr Ser Val Thr Val Ser Leu
        115             120
```

```
<210>  10
<211>  124
<212>  PRT
<213>  Mus musculus

<400>  10
```

```
Gln Ile Gln Leu Val Gln Ser Gly Pro Glu Leu Lys Lys Pro Gly Glu
1               5               10              15
```

Thr Val Lys Ile Ser Cys Lys Ala Ser Gly Tyr Thr Phe Thr Asn Phe
                20              25              30

Gly Val Asn Trp Val Lys Gln Ala Pro Gly Lys Gly Leu Lys Trp Met
            35              40              45

Gly Trp Ile Asn Thr Tyr Thr Gly Glu Pro Ser Tyr Ala Asp Asp Phe
        50              55              60

Lys Gly Arg Phe Ala Phe Ser Leu Glu Ala Ser Ala Asn Thr Ala Tyr
65              70              75              80

Leu Gln Ile Asn Asn Leu Lys Asn Asp Asp Met Ser Thr Tyr Phe Cys
                85              90              95

Thr Arg Arg Thr Asn Tyr Tyr Gly Thr Ser Tyr Tyr Tyr Ala Met Asp
            100             105             110

Tyr Trp Gly Gln Gly Thr Ser Val Thr Val Ser Ser
        115             120

<210> 11
<211> 119
<212> PRT
<213> Mus musculus

<400> 11

Gln Val Gln Leu Gln Gln Pro Gly Ala Glu Leu Val Lys Pro Gly Ala
1               5               10              15

Ser Val Lys Met Ser Cys Lys Ala Ser Gly Tyr Thr Phe Thr Ser Tyr
                20              25              30

Asn Ile His Trp Val Lys Gln Thr Pro Gly Gln Gly Leu Glu Trp Ile
            35              40              45

Gly Ala Ile Tyr Pro Gly Asn Gly Asp Thr Ser Tyr Asn Gln Lys Phe
        50              55              60

Lys Gly Lys Ala Thr Leu Thr Ser Asp Lys Ser Ser Ser Thr Ala Tyr
65              70              75              80

Met Gln Leu Ser Ser Leu Thr Ser Glu Asp Ser Ala Val Tyr Tyr Cys
                85              90              95

Ala Arg Met Ser Thr Met Ile Thr Gly Phe Asp Tyr Trp Gly Gln Gly
            100             105             110

Thr Thr Leu Thr Val Ser Ser
        115

```
<210>  12
<211>  122
<212>  PRT
<213>  Mus musculus

<400>  12

Gln Val Gln Leu Gln Gln Pro Gly Ala Glu Leu Val Lys Pro Gly Ala
1               5                   10                  15

Ser Val Lys Met Ser Cys Lys Ala Ser Gly Phe Thr Phe Thr Ser Tyr
            20                  25                  30

Asn Leu His Trp Val Lys Gln Thr Pro Gly Gln Gly Leu Glu Trp Ile
        35                  40                  45

Gly Ala Ile Tyr Pro Gly Ser Gly Asp Thr Ser Tyr Asn Gln Gln Phe
    50                  55                  60

Lys Gly Lys Ala Thr Leu Thr Ala Asp Lys Ser Ser Asn Thr Ala Tyr
65                  70                  75                  80

Met Gln Leu Asn Ser Leu Thr Ser Glu Asp Ser Ala Val Tyr Cys Cys
                85                  90                  95

Ala Arg Ser Ala Met Ile Ser Thr Gly Asn Trp Tyr Phe Asp Tyr Trp
            100                 105                 110

Gly Gln Gly Thr Thr Leu Thr Val Ser Ser
            115                 120


<210>  13
<211>  121
<212>  PRT
<213>  Mus musculus

<400>  13

Gln Val Gln Leu Gln Gln Pro Gly Ala Glu Leu Val Lys Pro Gly Ala
1               5                   10                  15

Ser Val Lys Met Ser Cys Lys Ala Ser Gly Phe Thr Phe Thr Ser Tyr
            20                  25                  30

Asn Met His Trp Val Lys Gln Thr Pro Gly Gln Gly Leu Glu Trp Ile
        35                  40                  45

Gly Gly Ile Tyr Pro Gly Asn Gly Asp Thr Ser Tyr Asn Gln Lys Phe
    50                  55                  60

Lys Gly Lys Ala Thr Leu Thr Ala Asp Lys Ser Ser Ser Thr Ala Tyr
65                  70                  75                  80

Met Gln Leu Ser Ser Leu Thr Ser Glu Asp Ser Ala Val Tyr Tyr Cys
```

```
                         85                    90                    95

     Ala Arg Phe Tyr Tyr Tyr Gly Ser Met Gly Ala Met Asp Tyr Trp Gly
                 100             105             110

     Gln Gly Thr Ser Val Thr Val Ser Ser
                 115             120


     <210>  14
     <211>  122
     <212>  PRT
     <213>  Mus musculus

     <400>  14

     Gln Val Gln Leu Gln Gln Pro Gly Ala Glu Leu Val Lys Pro Gly Ala
     1               5               10              15

     Ser Val Lys Met Ser Cys Lys Ala Ser Gly Tyr Thr Phe Thr Thr Tyr
                 20              25              30

     Asn Leu His Trp Val Lys Gln Thr Pro Gly Gln Gly Leu Glu Trp Ile
                 35              40              45

     Gly Gly Ile Tyr Pro Gly Asn Gly Asp Thr Ser Tyr Asn Gln Lys Phe
             50              55              60

     Lys Val Lys Ala Thr Leu Thr Ala Asp Lys Ser Ser Asn Thr Ala Tyr
     65              70              75              80

     Met Gln Leu Ser Ser Leu Thr Ser Glu Asp Ser Ala Val Tyr Tyr Cys
                 85              90              95

     Ala Arg Trp Ile Tyr Tyr Gly Asn Tyr Glu Gly Thr Leu Asp Tyr Trp
                 100             105             110

     Gly Gln Gly Thr Ser Val Thr Val Ser Ser
                 115             120


     <210>  15
     <211>  116
     <212>  PRT
     <213>  Mus musculus

     <400>  15

     Gln Val Gln Leu Gln Gln Ser Gly Ala Glu Leu Ala Lys Pro Gly Ala
     1               5               10              15

     Ser Val Lys Met Ser Cys Lys Ala Ser Gly Tyr Thr Phe Thr Ser Tyr
                 20              25              30

     Trp Met His Trp Val Lys Gln Arg Pro Gly Gln Gly Leu Glu Trp Ile
                 35              40              45
```

```
Gly Tyr Ile Thr Pro Ser Thr Gly Tyr Thr Asp Tyr Asn Lys Lys Phe
        50              55              60

Lys Asp Lys Ala Thr Leu Thr Ala Asp Arg Ser Ser Ser Thr Ala Tyr
65              70              75              80

Met His Leu Ser Ser Leu Thr Ser Glu Asp Ser Ala Val Tyr Tyr Cys
                85              90              95

Ala Arg Ser Gly Pro Tyr Phe Asp Val Trp Gly Ala Gly Thr Thr Val
            100             105             110

Thr Val Ser Ser
        115
```

```
<210>  16
<211>  119
<212>  PRT
<213>  Mus musculus

<400>  16
```

```
Gln Val Gln Leu Gln Gln Pro Gly Ala Glu Leu Val Lys Pro Gly Ala
1               5               10              15

Ser Val Lys Val Ser Cys Lys Ala Ser Gly Phe Thr Phe Thr Ser Tyr
        20              25              30

Asn Leu His Trp Val Lys Gln Thr Pro Gly Gln Gly Leu Val Trp Ile
        35              40              45

Gly Ala Ile Tyr Pro Gly Asn Gly Asp Thr Ser Tyr Asn Gln Lys Phe
        50              55              60

Arg Gly Lys Ala Thr Leu Thr Ala Asp Ile Ser Ser Ser Thr Ala Tyr
65              70              75              80

Met Gln Leu Ser Ser Leu Thr Ser Glu Asp Ser Ala Val Tyr Tyr Cys
                85              90              95

Ala Arg Tyr Tyr Tyr Gly Tyr Asp Ala Met Asp Tyr Trp Gly Gln Gly
            100             105             110

Thr Ser Val Thr Val Ser Ser
        115
```

```
<210>  17
<211>  107
<212>  PRT
<213>  Artificial

<220>
<223>  L chain V region sequence of humanized antibody abb 1791
```

<400> 17

```
Ser Thr Val Met Thr Gln Ser Pro Asp Ser Leu Ala Val Ser Leu Gly
1               5                   10                  15

Glu Arg Ala Thr Ile Asn Cys Lys Ser Ser Gln Ser Val Ser Asn Asp
            20              25              30

Val Ala Trp Tyr Gln Gln Lys Pro Gly Gln Ser Pro Lys Val Leu Ile
        35              40              45

Tyr Phe Ala Ser Asn Arg Tyr Ser Gly Val Pro Asp Arg Phe Ser Gly
    50              55              60

Ser Gly Tyr Gly Thr Asp Phe Thr Leu Thr Ile Ser Ser Leu Gln Ala
65              70              75              80

Glu Asp Val Ala Val Tyr Phe Cys Gln Gln Asp Tyr Ser Ser Pro Leu
            85              90              95

Thr Phe Gly Ala Gly Thr Lys Leu Glu Ile Lys
            100             105
```

<210> 18
<211> 107
<212> PRT
<213> Artificial

<220>
<223> L chain V region sequence of humanized antibody fra 1791

<400> 18

```
Ser Thr Val Met Thr Gln Ser Pro Ser Phe Leu Ser Ala Ser Val Gly
1               5                   10                  15

Asp Arg Val Thr Ile Thr Cys Lys Ala Ser Gln Ser Val Ser Asn Asp
            20              25              30

Val Ala Trp Tyr Gln Gln Lys Pro Gly Gln Ser Pro Lys Val Leu Ile
        35              40              45

Tyr Phe Ala Ser Asn Arg Tyr Thr Gly Val Pro Asp Arg Phe Ser Gly
    50              55              60

Ser Gly Tyr Gly Thr Asp Phe Thr Leu Thr Ile Ser Ser Leu Gln Ala
65              70              75              80

Glu Asp Val Ala Val Tyr Phe Cys Gln Gln Asp Tyr Ser Ser Pro Leu
            85              90              95

Thr Phe Gly Ala Gly Thr Lys Leu Glu Ile Lys
            100             105
```

```
<210>  19
<211>  107
<212>  PRT
<213>  Artificial

<220>
<223>  L chain V region sequence of humanized antibody sdr 1791

<400>  19

Ser Thr Val Met Thr Gln Ser Pro Asp Ser Leu Ala Val Ser Leu Gly
1               5                   10                  15

Glu Arg Ala Thr Ile Asn Cys Lys Ser Ser Gln Ser Asn Ser Asn Asp
                20                  25                  30

Val Ala Trp Tyr Gln Gln Lys Pro Gly Gln Ser Pro Lys Val Leu Ile
            35                  40                  45

Tyr Phe Ala Ser Asn Arg Tyr Ser Gly Val Pro Asp Arg Phe Ser Gly
        50                  55                  60

Ser Gly Tyr Gly Thr Asp Phe Thr Leu Thr Ile Ser Ser Leu Gln Ala
65                  70                  75                  80

Glu Asp Val Ala Val Tyr Phe Cys Gln Gln Asp Tyr Ser Ser Pro Leu
                85                  90                  95

Thr Phe Gly Ala Gly Thr Lys Leu Glu Ile Lys
            100                 105


<210>  20
<211>  107
<212>  PRT
<213>  Artificial

<220>
<223>  L chain V region sequence of humanized antibody Ven 1791

<400>  20

Ser Thr Val Met Thr Gln Ser Pro Asp Ser Leu Ala Val Ser Leu Gly
1               5                   10                  15

Glu Arg Val Thr Ile Asn Cys Lys Ala Ser Gln Ser Val Ser Asn Asp
                20                  25                  30

Val Ala Trp Tyr Gln Gln Lys Pro Gly Gln Ser Pro Lys Val Leu Ile
            35                  40                  45

Tyr Phe Ala Ser Asn Arg Tyr Thr Gly Val Pro Asp Arg Phe Ser Gly
        50                  55                  60

Ser Gly Tyr Gly Thr Asp Phe Thr Phe Thr Ile Ser Ser Val Gln Ala
65                  70                  75                  80
```

```
Glu Asp Val Ala Val Tyr Phe Cys Gln Gln Asp Tyr Ser Ser Pro Leu
                85                  90                  95

Thr Phe Gly Ala Gly Thr Lys Leu Glu Leu Lys
            100                 105


<210>   21
<211>   124
<212>   PRT
<213>   Artificial

<220>
<223>   H chain V region sequence of humanized antibody abb 1791

<400>   21

Gln Ile Gln Leu Val Gln Ser Gly Ser Glu Leu Lys Lys Pro Gly Ala
1               5                   10                  15

Ser Val Lys Val Ser Cys Lys Ala Ser Gly Tyr Thr Phe Thr Asn Phe
                20                  25                  30

Gly Val Asn Trp Val Arg Gln Ala Pro Gly Lys Gly Leu Glu Trp Met
            35                  40                  45

Gly Trp Ile Asn Thr Tyr Thr Gly Glu Pro Ser Tyr Ala Gln Gly Phe
    50                  55                  60

Thr Gly Arg Phe Val Phe Ser Leu Asp Ala Ser Val Ser Thr Ala Tyr
65                  70                  75                  80

Leu Gln Ile Ser Ser Leu Lys Ala Glu Asp Thr Ala Thr Tyr Phe Cys
                85                  90                  95

Thr Arg Arg Thr Asn Tyr Tyr Gly Thr Ser Tyr Tyr Tyr Ala Met Asp
            100                 105                 110

Tyr Trp Gly Gln Gly Thr Thr Val Thr Val Ser Ser
            115                 120


<210>   22
<211>   124
<212>   PRT
<213>   Artificial

<220>
<223>   H chain V region sequence of humanized antibody fra 1791

<400>   22

Gln Ile Gln Leu Val Gln Ser Gly Ser Glu Leu Lys Lys Pro Gly Ala
1               5                   10                  15

Ser Val Lys Val Ser Cys Lys Ala Ser Gly Tyr Thr Phe Thr Asn Phe
                20                  25                  30
```

39

```
Gly Val Asn Trp Val Lys Gln Ala Pro Gly Lys Gly Leu Lys Trp Met
        35                  40                  45

Gly Trp Ile Asn Thr Tyr Thr Gly Glu Pro Ser Tyr Ala Asp Asp Phe
        50                  55                  60

Lys Gly Arg Phe Ala Phe Ser Leu Asp Ala Ser Ala Ser Thr Ala Tyr
65                  70                  75                  80

Leu Gln Ile Ser Ser Leu Lys Ala Glu Asp Met Ala Thr Tyr Phe Cys
                85                  90                  95

Thr Arg Arg Thr Asn Tyr Tyr Gly Thr Ser Tyr Tyr Tyr Ala Met Asp
            100                 105                 110

Tyr Trp Gly Gln Gly Thr Thr Val Thr Val Ser Ser
        115                 120
```

```
<210>   23
<211>   124
<212>   PRT
<213>   Artificial

<220>
<223>   H chain V region sequence of humanized antibody sdr 1791

<400>   23
```

```
Gln Ile Gln Leu Val Gln Ser Gly Ser Glu Leu Lys Lys Pro Gly Ala
1               5                   10                  15

Ser Val Lys Val Ser Cys Lys Ala Ser Gly Tyr Thr Phe Thr Asn Phe
            20                  25                  30

Gly Val Asn Trp Val Arg Gln Ala Pro Gly Lys Gly Leu Lys Trp Met
        35                  40                  45

Gly Trp Ile Asn Thr Tyr Thr Gly Glu Pro Ser Tyr Ala Gln Gly Phe
        50                  55                  60

Thr Gly Arg Phe Ala Phe Ser Leu Asp Ala Ser Val Ser Thr Ala Tyr
65                  70                  75                  80

Leu Gln Ile Ser Ser Leu Lys Ala Glu Asp Thr Ala Thr Tyr Phe Cys
                85                  90                  95

Thr Arg Arg Thr Asn Tyr Tyr Gly Thr Ser Tyr Tyr Tyr Ala Met Asp
            100                 105                 110

Tyr Trp Gly Gln Gly Thr Thr Val Thr Val Ser Ser
        115                 120
```

```
<210>   24
<211>   124
```

```
<212>   PRT
<213>   Artificial

<220>
<223>   H chain V region sequence of humanized antibody Ven 1791

<400>   24
```

Gln Ile Gln Leu Val Gln Ser Gly Pro Glu Leu Lys Lys Pro Gly Ala
1                5                10                15

Ser Val Lys Ile Ser Cys Lys Ala Ser Gly Tyr Thr Phe Thr Asn Phe
                20              25              30

Gly Val Asn Trp Val Lys Gln Ala Pro Gly Lys Gly Leu Lys Trp Met
            35              40              45

Gly Trp Ile Asn Thr Tyr Thr Gly Glu Pro Ser Tyr Ala Asp Asp Phe
    50              55              60

Lys Gly Arg Phe Ala Phe Ser Leu Asp Ala Ser Val Ser Thr Ala Tyr
65              70              75              80

Leu Gln Ile Ser Ser Leu Lys Ala Glu Asp Thr Ser Thr Tyr Phe Cys
                85              90              95

Thr Arg Arg Thr Asn Tyr Tyr Gly Thr Ser Tyr Tyr Tyr Ala Met Asp
            100             105             110

Tyr Trp Gly Gln Gly Thr Thr Val Thr Val Ser Ser
        115             120

```
<210>   25
<211>   35
<212>   DNA
<213>   Artificial sequence

<220>
<223>   primer

<400>   25
```

aatgcggccg ccaccatgac aacacccaga aattc                                    35

```
<210>   26
<211>   29
<212>   DNA
<213>   Artificial sequence

<220>
<223>   primer

<400>   26
```

gctctagatt aaggagagct gtcattttc                                           29

SEQUENCE LISTING

<110>  OSAKA UNIVERSITY

<120>  Humanized anti CD20 monoclonal antibody

<130>  666838

<150>  PCT/JP2006/304370
<150>  2006-03-07

<160>  26

<170>  PatentIn version 3.2

<210>  1
<211>  106
<212>  PRT
<213>  Mus musculus

<400>  1

Gln Ile Val Leu Thr Gln Ser Pro Pro Ile Met Ser Ala Ser Leu Gly
1               5                   10                  15

Glu Glu Ile Thr Leu Thr Cys Ser Ala Ser Ser Arg Val Ser Tyr Met
            20                  25                  30

Leu Trp Tyr Gln Gln Lys Ser Gly Thr Ser Pro Lys Leu Leu Ile Tyr
        35                  40                  45

Ser Thr Ser Asn Leu Ala Ser Gly Val Pro Ser Arg Phe Ser Gly Ser
    50                  55                  60

Gly Ser Gly Thr Phe Tyr Ser Leu Thr Ile Ser Ser Val Glu Ala Glu
65                  70                  75                  80

Asp Ala Ala Asp Tyr Tyr Cys His Gln Trp Thr Ser Asn Pro Cys Thr
                85                  90                  95

Phe Gly Gly Gly Thr Lys Leu Glu Ile Lys
            100                 105

<210>  2
<211>  107
<212>  PRT
<213>  Mus musculus

<400>  2

Ser Thr Val Met Thr Gln Thr Pro Lys Phe Leu Leu Val Ser Ala Gly
1               5                   10                  15

Asp Arg Val Thr Ile Thr Cys Lys Ala Ser Gln Ser Val Ser Asn Asp

42

Val Ala Trp Tyr Gln Gln Lys Pro Gly Gln Ser Pro Lys Val Leu Ile
35 40 45

Tyr Phe Ala Ser Asn Arg Tyr Thr Gly Val Pro Asp Arg Phe Thr Gly
50 55 60

Ser Gly Tyr Gly Thr Asp Phe Thr Phe Thr Ile Asn Thr Val Gln Ala
65 70 75 80

Glu Asp Leu Ala Val Tyr Phe Cys Gln Gln Asp Tyr Ser Ser Pro Leu
85 90 95

Thr Phe Gly Ala Gly Thr Lys Leu Glu Leu Lys
100 105

<210> 3
<211> 106
<212> PRT
<213> Mus musculus

<400> 3

Gln Ile Val Leu Ser Gln Ser Pro Ala Ile Leu Ser Ala Ser Pro Gly
1 5 10 15

Glu Lys Val Thr Met Thr Cys Arg Ala Ser Ser Ser Val Ser Tyr Met
20 25 30

His Trp Tyr Gln Gln Arg Pro Gly Ser Ser Pro Lys Pro Trp Ile Tyr
35 40 45

Ala Thr Ser Asn Leu Ala Ser Gly Val Pro Ala Arg Phe Ser Gly Ser
50 55 60

Gly Ser Gly Thr Ser Tyr Tyr Phe Thr Ile Ser Arg Val Glu Ala Glu
65 70 75 80

Asp Ala Ala Thr Tyr Tyr Cys Gln Gln Trp Asn Ser Asn Pro Pro Thr
85 90 95

His Gly Gly Gly Thr Lys Leu Glu Ile Lys
100 105

<210> 4
<211> 106
<212> PRT
<213> Mus musculus

<400> 4

Gln Ile Val Leu Ser Gln Ser Pro Ala Ile Leu Ser Ala Ser Pro Gly
1               5                   10                  15

Glu Lys Val Thr Met Thr Cys Arg Ala Ser Ser Ser Val Ser Tyr Met
                20                  25                  30

Asp Trp Tyr Gln Gln Lys Pro Gly Ser Ser Pro Lys Pro Trp Ile Tyr
            35                  40                  45

Ala Thr Ser Asn Leu Ala Ser Gly Val Pro Ala Arg Phe Ser Gly Ser
            50                  55                  60

Gly Ser Gly Thr Ser Tyr Ser Leu Thr Ile Ser Arg Val Glu Ala Glu
65                  70                  75                  80

Asp Thr Ala Thr Tyr Tyr Cys Gln Gln Trp Thr Phe Asn Pro Pro Thr
                85                  90                  95

Phe Gly Ser Gly Thr Lys Leu Glu Ile Lys
            100                 105

<210> 5
<211> 106
<212> PRT
<213> Mus musculus

<400> 5

Gln Ile Val Leu Ser Gln Ser Pro Ala Ile Leu Ser Ala Ser Pro Gly
1               5                   10                  15

Glu Lys Val Thr Met Thr Cys Arg Ala Ser Ser Ser Val Ser Tyr Met
                20                  25                  30

His Trp Tyr Gln Gln Lys Pro Gly Ser Ser Pro Lys Pro Trp Ile Tyr
            35                  40                  45

Ala Thr Ser Asn Leu Ala Ser Gly Val Pro Ala Arg Phe Ser Gly Ser
            50                  55                  60

Gly Ser Gly Thr Ser Tyr Ser Leu Thr Ile Thr Arg Val Glu Ala Glu
65                  70                  75                  80

Asp Ala Ala Thr Tyr Tyr Cys Gln Gln Trp Thr Phe Asn Pro Pro Thr
                85                  90                  95

Phe Gly Ala Gly Thr Lys Leu Glu Leu Lys
100              105

<210> 6
<211> 106
<212> PRT
<213> Mus musculus

<400> 6

Gln Ile Val Leu Ser Gln Ser Pro Ala Ile Leu Ser Ser Ser Pro Gly
1              5              10              15

Glu Lys Val Thr Met Thr Cys Arg Ala Ser Ser Ser Val Ser Tyr Met
20              25              30

Leu Trp Tyr Gln Gln Lys Pro Gly Ser Ser Pro Glu Pro Trp Ile Tyr
35              40              45

Ala Thr Ser Asn Leu Ala Ser Gly Val Pro Ala Arg Phe Ser Gly Gly
50              55              60

Gly Ser Gly Thr Ser Tyr Ser Leu Thr Ile Ser Arg Val Glu Ala Glu
65              70              75              80

Asp Ala Ala Thr Tyr Tyr Cys Gln Gln Trp Thr Phe Asn Pro Pro Thr
85              90              95

Phe Gly Gly Gly Thr Lys Leu Glu Ile Lys
100              105

<210> 7
<211> 107
<212> PRT
<213> Mus musculus

<400> 7

Asp Ile Leu Leu Thr Gln Ser Pro Ala Ile Leu Phe Val Ser Pro Gly
1              5              10              15

Glu Arg Val Ser Leu Ser Cys Arg Ala Ser Gln Asn Ile Gly Thr Ser
20              25              30

Ile His Trp Tyr Gln Gln Arg Thr Asn Gly Ser Pro Arg Leu Leu Ile
35              40              45

Lys Tyr Ala Ser Glu Ser Phe Ser Gly Ile Pro Ser Arg Phe Ser Gly
50              55              60

Ser Gly Ser Gly Thr Asp Phe Thr Leu Ser Ile Asn Ser Val Glu Ser
65              70              75              80

Glu Asp Ile Ala Asp Tyr Tyr Cys Gln Gln Ser Asn Ser Trp Pro Phe
85              90              95

Thr Phe Gly Ser Gly Thr Lys Leu Glu Ile Lys
          100                105

<210> 8
<211> 108
<212> PRT
<213> Mus musculus

<400> 8

Glu Ile Ile Leu Thr Gln Ser Pro Thr Thr Met Ala Ala Ser Pro Gly
1             5                 10               15

Glu Lys Ile Thr Ile Thr Cys Ser Ala Ser Ser Ser Ile Ser Ser Tyr
          20                25               30

Tyr Leu Arg Trp Tyr Gln Gln Lys Pro Gly Phe Ser Pro Lys Val Leu
          35                40               45

Ile Tyr Arg Thr Ser Asn Leu Ala Ser Gly Val Pro Ala Arg Phe Ser
          50                55               60

Gly Ser Gly Ser Gly Thr Ser Tyr Ser Leu Thr Ile Gly Thr Met Glu
65                 70               75               80

Ala Glu Asp Val Ala Thr Tyr Tyr Cys Gln Gln Gly Asn Thr Val Pro
          85                90               95

Leu Thr Phe Gly Ser Gly Thr Lys Leu Glu Ile Lys
          100                105

<210> 9
<211> 123
<212> PRT
<213> Mus musculus

<400> 9

Gln Val Gln Leu Gln Gln Pro Gly Ala Glu Leu Val Lys Pro Gly Ala
1             5                 10               15

Ser Val Lys Met Ser Cys Arg Ala Ser Gly Tyr Thr Phe Thr Asn Tyr
          20                25               30

Asn Met His Trp Ile Lys Gln Thr Pro Gly Gln Gly Leu Glu Trp Ile
          35                40               45

Gly Ala Ile Tyr Pro Gly Ser Gly Asp Thr Ser Tyr Asn Arg Lys Phe
          50                55               60

```
Lys Gly Lys Ala Thr Leu Thr Ala Asp Thr Ser Ser Ser Thr Ala Tyr
65              70              75              80

Met Gln Phe Ser Ser Leu Thr Ser Ala Asp Ser Ala Val Tyr Tyr Cys
            85              90              95

Ala Arg Phe Thr Tyr Tyr Tyr Gly Gly Thr Tyr Gly Ala Met Asp Tyr
            100             105             110

Trp Gly Gln Gly Thr Ser Val Thr Val Ser Leu
            115             120
```

```
<210>   10
<211>   124
<212>   PRT
<213>   Mus musculus

<400>   10
```

```
Gln Ile Gln Leu Val Gln Ser Gly Pro Glu Leu Lys Lys Pro Gly Glu
1               5               10              15

Thr Val Lys Ile Ser Cys Lys Ala Ser Gly Tyr Thr Phe Thr Asn Phe
            20              25              30

Gly Val Asn Trp Val Lys Gln Ala Pro Gly Lys Gly Leu Lys Trp Met
            35              40              45

Gly Trp Ile Asn Thr Tyr Thr Gly Glu Pro Ser Tyr Ala Asp Asp Phe
    50              55              60

Lys Gly Arg Phe Ala Phe Ser Leu Glu Ala Ser Ala Asn Thr Ala Tyr
    65              70              75              80

Leu Gln Ile Asn Asn Leu Lys Asn Asp Asp Met Ser Thr Tyr Phe Cys
            85              90              95

Thr Arg Arg Thr Asn Tyr Tyr Gly Thr Ser Tyr Tyr Tyr Ala Met Asp
            100             105             110

Tyr Trp Gly Gln Gly Thr Ser Val Thr Val Ser Ser
            115             120
```

```
<210>   11
<211>   119
<212>   PRT
<213>   Mus musculus
```

<400> 11

Gln Val Gln Leu Gln Gln Pro Gly Ala Glu Leu Val Lys Pro Gly Ala
1               5                   10                  15

Ser Val Lys Met Ser Cys Lys Ala Ser Gly Tyr Thr Phe Thr Ser Tyr
                20                  25                  30

Asn Ile His Trp Val Lys Gln Thr Pro Gly Gln Gly Leu Glu Trp Ile
            35                  40                  45

Gly Ala Ile Tyr Pro Gly Asn Gly Asp Thr Ser Tyr Asn Gln Lys Phe
        50                  55                  60

Lys Gly Lys Ala Thr Leu Thr Ser Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Arg Met Ser Thr Met Ile Thr Gly Phe Asp Tyr Trp Gly Gln Gly
                100                 105                 110

Thr Thr Leu Thr Val Ser Ser
            115

<210> 12
<211> 122
<212> PRT
<213> Mus musculus

<400> 12

Gln Val Gln Leu Gln Gln Pro Gly Ala Glu Leu Val Lys Pro Gly Ala
1               5                   10                  15

Ser Val Lys Met Ser Cys Lys Ala Ser Gly Phe Thr Phe Thr Ser Tyr
                20                  25                  30

Asn Leu His Trp Val Lys Gln Thr Pro Gly Gln Gly Leu Glu Trp Ile
            35                  40                  45

Gly Ala Ile Tyr Pro Gly Ser Gly Asp Thr Ser Tyr Asn Gln Gln Phe
        50                  55                  60

Lys Gly Lys Ala Thr Leu Thr Ala Asp Lys Ser Ser Asn Thr Ala Tyr
65                  70                  75                  80

Met Gln Leu Asn Ser Leu Thr Ser Glu Asp Ser Ala Val Tyr Cys Cys

                    85                      90                      95

Ala Arg Ser Ala Met Ile Ser Thr Gly Asn Trp Tyr Phe Asp Tyr Trp
                100                     105                     110

Gly Gln Gly Thr Thr Leu Thr Val Ser Ser
                115                     120


<210>  13
<211>  121
<212>  PRT
<213>  Mus musculus

<400>  13

Gln Val Gln Leu Gln Gln Pro Gly Ala Glu Leu Val Lys Pro Gly Ala
1               5                   10                  15

Ser Val Lys Met Ser Cys Lys Ala Ser Gly Phe Thr Phe Thr Ser Tyr
                20                  25                  30

Asn Met His Trp Val Lys Gln Thr Pro Gly Gln Gly Leu Glu Trp Ile
            35                  40                  45

Gly Gly Ile Tyr Pro Gly Asn Gly Asp Thr Ser Tyr Asn Gln Lys Phe
        50                  55                  60

Lys Gly Lys Ala Thr Leu Thr Ala Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Arg Phe Tyr Tyr Tyr Gly Ser Met Gly Ala Met Asp Tyr Trp Gly
                100                     105                     110

Gln Gly Thr Ser Val Thr Val Ser Ser
                115                     120


<210>  14
<211>  122
<212>  PRT
<213>  Mus musculus

<400>  14

Gln Val Gln Leu Gln Gln Pro Gly Ala Glu Leu Val Lys Pro Gly Ala
1               5                   10                  15

Ser Val Lys Met Ser Cys Lys Ala Ser Gly Tyr Thr Phe Thr Thr Tyr

```
                    20                  25                  30

Asn Leu His Trp Val Lys Gln Thr Pro Gly Gln Gly Leu Glu Trp Ile
        35                  40                  45

Gly Gly Ile Tyr Pro Gly Asn Gly Asp Thr Ser Tyr Asn Gln Lys Phe
        50                  55                  60

Lys Val Lys Ala Thr Leu Thr Ala Asp Lys Ser Ser Asn Thr Ala Tyr
65                  70                  75                  80

Met Gln Leu Ser Ser Leu Thr Ser Glu Asp Ser Ala Val Tyr Tyr Cys
                85                  90                  95

Ala Arg Trp Ile Tyr Tyr Gly Asn Tyr Glu Gly Thr Leu Asp Tyr Trp
                100                 105                 110

Gly Gln Gly Thr Ser Val Thr Val Ser Ser
            115                 120
```

```
<210>  15
<211>  116
<212>  PRT
<213>  Mus musculus

<400>  15
```

```
Gln Val Gln Leu Gln Gln Ser Gly Ala Glu Leu Ala Lys Pro Gly Ala
1               5                   10                  15

Ser Val Lys Met Ser Cys Lys Ala Ser Gly Tyr Thr Phe Thr Ser Tyr
            20                  25                  30

Trp Met His Trp Val Lys Gln Arg Pro Gly Gln Gly Leu Glu Trp Ile
        35                  40                  45

Gly Tyr Ile Thr Pro Ser Thr Gly Tyr Thr Asp Tyr Asn Lys Lys Phe
        50                  55                  60

Lys Asp Lys Ala Thr Leu Thr Ala Asp Arg Ser Ser Ser Thr Ala Tyr
65                  70                  75                  80

Met His Leu Ser Ser Leu Thr Ser Glu Asp Ser Ala Val Tyr Tyr Cys
                85                  90                  95

Ala Arg Ser Gly Pro Tyr Phe Asp Val Trp Gly Ala Gly Thr Thr Val
            100                 105                 110
```

Thr Val Ser Ser
          115

<210> 16
<211> 119
<212> PRT
<213> Mus musculus

<400> 16

Gln Val Gln Leu Gln Gln Pro Gly Ala Glu Leu Val Lys Pro Gly Ala
1               5                   10                  15

Ser Val Lys Val Ser Cys Lys Ala Ser Gly Phe Thr Phe Thr Ser Tyr
              20                  25                  30

Asn Leu His Trp Val Lys Gln Thr Pro Gly Gln Gly Leu Val Trp Ile
          35                  40                  45

Gly Ala Ile Tyr Pro Gly Asn Gly Asp Thr Ser Tyr Asn Gln Lys Phe
      50                  55                  60

Arg Gly Lys Ala Thr Leu Thr Ala Asp Ile Ser Ser Ser Thr Ala Tyr
65                  70                  75                  80

Met Gln Leu Ser Ser Leu Thr Ser Glu Asp Ser Ala Val Tyr Tyr Cys
              85                  90                  95

Ala Arg Tyr Tyr Tyr Gly Tyr Asp Ala Met Asp Tyr Trp Gly Gln Gly
          100                 105                 110

Thr Ser Val Thr Val Ser Ser
          115

<210> 17
<211> 107
<212> PRT
<213> Artificial

<220>
<223> L chain V region sequence of humanized antibody abb 1791

<400> 17

Ser Thr Val Met Thr Gln Ser Pro Asp Ser Leu Ala Val Ser Leu Gly
1               5                   10                  15

Glu Arg Ala Thr Ile Asn Cys Lys Ser Ser Gln Ser Val Ser Asn Asp
              20                  25                  30

Val Ala Trp Tyr Gln Gln Lys Pro Gly Gln Ser Pro Lys Val Leu Ile

```
                    35                   40                   45

Tyr Phe Ala Ser Asn Arg Tyr Ser Gly Val Pro Asp Arg Phe Ser Gly
        50                   55                   60

Ser Gly Tyr Gly Thr Asp Phe Thr Leu Thr Ile Ser Ser Leu Gln Ala
65                   70                   75                   80

Glu Asp Val Ala Val Tyr Phe Cys Gln Gln Asp Tyr Ser Ser Pro Leu
                85                   90                   95

Thr Phe Gly Ala Gly Thr Lys Leu Glu Ile Lys
                100                  105


<210>  18
<211>  107
<212>  PRT
<213>  Artificial

<220>
<223>  L chain V region sequence of humanized antibody fra 1791

<400>  18

Ser Thr Val Met Thr Gln Ser Pro Ser Phe Leu Ser Ala Ser Val Gly
1                5                   10                   15

Asp Arg Val Thr Ile Thr Cys Lys Ala Ser Gln Ser Val Ser Asn Asp
           20                   25                   30

Val Ala Trp Tyr Gln Gln Lys Pro Gly Gln Ser Pro Lys Val Leu Ile
        35                   40                   45

Tyr Phe Ala Ser Asn Arg Tyr Thr Gly Val Pro Asp Arg Phe Ser Gly
        50                   55                   60

Ser Gly Tyr Gly Thr Asp Phe Thr Leu Thr Ile Ser Ser Leu Gln Ala
65                   70                   75                   80

Glu Asp Val Ala Val Tyr Phe Cys Gln Gln Asp Tyr Ser Ser Pro Leu
                85                   90                   95

Thr Phe Gly Ala Gly Thr Lys Leu Glu Ile Lys
                100                  105


<210>  19
<211>  107
<212>  PRT
<213>  Artificial
```

<220>
<223> L chain V region sequence of humanized antibody sdr 1791

<400> 19

Ser Thr Val Met Thr Gln Ser Pro Asp Ser Leu Ala Val Ser Leu Gly
1               5               10              15

Glu Arg Ala Thr Ile Asn Cys Lys Ser Ser Gln Ser Asn Ser Asn Asp
                20              25              30

Val Ala Trp Tyr Gln Gln Lys Pro Gly Gln Ser Pro Lys Val Leu Ile
            35              40              45

Tyr Phe Ala Ser Asn Arg Tyr Ser Gly Val Pro Asp Arg Phe Ser Gly
    50              55              60

Ser Gly Tyr Gly Thr Asp Phe Thr Leu Thr Ile Ser Ser Leu Gln Ala
65              70              75              80

Glu Asp Val Ala Val Tyr Phe Cys Gln Gln Asp Tyr Ser Ser Pro Leu
            85              90              95

Thr Phe Gly Ala Gly Thr Lys Leu Glu Ile Lys
            100             105

<210> 20
<211> 107
<212> PRT
<213> Artificial

<220>
<223> L chain V region sequence of humanized antibody Ven 1791

<400> 20

Ser Thr Val Met Thr Gln Ser Pro Asp Ser Leu Ala Val Ser Leu Gly
1               5               10              15

Glu Arg Val Thr Ile Asn Cys Lys Ala Ser Gln Ser Val Ser Asn Asp
                20              25              30

Val Ala Trp Tyr Gln Gln Lys Pro Gly Gln Ser Pro Lys Val Leu Ile
            35              40              45

Tyr Phe Ala Ser Asn Arg Tyr Thr Gly Val Pro Asp Arg Phe Ser Gly
    50              55              60

Ser Gly Tyr Gly Thr Asp Phe Thr Phe Thr Ile Ser Ser Val Gln Ala
65              70              75              80

Glu Asp Val Ala Val Tyr Phe Cys Gln Gln Asp Tyr Ser Ser Pro Leu
                85                  90                  95

Thr Phe Gly Ala Gly Thr Lys Leu Glu Leu Lys
            100                 105

<210> 21
<211> 124
<212> PRT
<213> Artificial

<220>
<223> H chain V region sequence of humanized antibody abb 1791

<400> 21

Gln Ile Gln Leu Val Gln Ser Gly Ser Glu Leu Lys Lys Pro Gly Ala
1               5                   10                  15

Ser Val Lys Val Ser Cys Lys Ala Ser Gly Tyr Thr Phe Thr Asn Phe
            20                  25                  30

Gly Val Asn Trp Val Arg Gln Ala Pro Gly Lys Gly Leu Glu Trp Met
            35                  40                  45

Gly Trp Ile Asn Thr Tyr Thr Gly Glu Pro Ser Tyr Ala Gln Gly Phe
        50                  55                  60

Thr Gly Arg Phe Val Phe Ser Leu Asp Ala Ser Val Ser Thr Ala Tyr
65                  70                  75                  80

Leu Gln Ile Ser Ser Leu Lys Ala Glu Asp Thr Ala Thr Tyr Phe Cys
                85                  90                  95

Thr Arg Arg Thr Asn Tyr Tyr Gly Thr Ser Tyr Tyr Tyr Ala Met Asp
            100                 105                 110

Tyr Trp Gly Gln Gly Thr Thr Val Thr Val Ser Ser
            115                 120

<210> 22
<211> 124
<212> PRT
<213> Artificial

<220>
<223> H chain V region sequence of humanized antibody fra 1791

<400> 22

Gln Ile Gln Leu Val Gln Ser Gly Ser Glu Leu Lys Lys Pro Gly Ala

Ser Val Lys Val Ser Cys Lys Ala Ser Gly Tyr Thr Phe Thr Asn Phe
20                    25                    30

Gly Val Asn Trp Val Lys Gln Ala Pro Gly Lys Gly Leu Lys Trp Met
        35                    40                    45

Gly Trp Ile Asn Thr Tyr Thr Gly Glu Pro Ser Tyr Ala Asp Asp Phe
    50                    55                    60

Lys Gly Arg Phe Ala Phe Ser Leu Asp Ala Ser Ala Ser Thr Ala Tyr
65                    70                    75                    80

Leu Gln Ile Ser Ser Leu Lys Ala Glu Asp Met Ala Thr Tyr Phe Cys
            85                    90                    95

Thr Arg Arg Thr Asn Tyr Tyr Gly Thr Ser Tyr Tyr Tyr Ala Met Asp
            100                   105                   110

Tyr Trp Gly Gln Gly Thr Thr Val Thr Val Ser Ser
        115                   120

<210> 23
<211> 124
<212> PRT
<213> Artificial

<220>
<223> H chain V region sequence of humanized antibody sdr 1791

<400> 23

Gln Ile Gln Leu Val Gln Ser Gly Ser Glu Leu Lys Lys Pro Gly Ala
1                 5                    10                    15

Ser Val Lys Val Ser Cys Lys Ala Ser Gly Tyr Thr Phe Thr Asn Phe
                20                    25                    30

Gly Val Asn Trp Val Arg Gln Ala Pro Gly Lys Gly Leu Lys Trp Met
        35                    40                    45

Gly Trp Ile Asn Thr Tyr Thr Gly Glu Pro Ser Tyr Ala Gln Gly Phe
    50                    55                    60

Thr Gly Arg Phe Ala Phe Ser Leu Asp Ala Ser Val Ser Thr Ala Tyr
65                    70                    75                    80

Leu Gln Ile Ser Ser Leu Lys Ala Glu Asp Thr Ala Thr Tyr Phe Cys

```
                    85                  90                  95

Thr Arg Arg Thr Asn Tyr Tyr Gly Thr Ser Tyr Tyr Tyr Ala Met Asp
            100                 105                 110

Tyr Trp Gly Gln Gly Thr Thr Val Thr Val Ser Ser
        115                 120
```

<210> 24
<211> 124
<212> PRT
<213> Artificial

<220>
<223> H chain V region sequence of humanized antibody Ven 1791

<400> 24

```
Gln Ile Gln Leu Val Gln Ser Gly Pro Glu Leu Lys Lys Pro Gly Ala
1               5                   10                  15

Ser Val Lys Ile Ser Cys Lys Ala Ser Gly Tyr Thr Phe Thr Asn Phe
            20                  25                  30

Gly Val Asn Trp Val Lys Gln Ala Pro Gly Lys Gly Leu Lys Trp Met
        35                  40                  45

Gly Trp Ile Asn Thr Tyr Thr Gly Glu Pro Ser Tyr Ala Asp Asp Phe
    50                  55                  60

Lys Gly Arg Phe Ala Phe Ser Leu Asp Ala Ser Val Ser Thr Ala Tyr
65                  70                  75                  80

Leu Gln Ile Ser Ser Leu Lys Ala Glu Asp Thr Ser Thr Tyr Phe Cys
                85                  90                  95

Thr Arg Arg Thr Asn Tyr Tyr Gly Thr Ser Tyr Tyr Tyr Ala Met Asp
            100                 105                 110

Tyr Trp Gly Gln Gly Thr Thr Val Thr Val Ser Ser
        115                 120
```

<210> 25
<211> 35
<212> DNA
<213> Artificial sequence

<220>
<223> primer

<400> 25

```
aatgcggccg ccaccatgac aacacccaga aattc                          35


<210>  26
<211>  29
<212>  DNA
<213>  Artificial sequence

<220>
<223>  primer

<400>  26

gctctagatt aaggagagct gtcattttc                                 29
```